# EUROPEAN PATENT SPECIFICATION

(11) **EP 2 451 398 B1**
(45) Date of publication and mention of the grant of the patent: **28.10.2015**
(21) Application number: 10797419.8
(22) Date of filing: 12.07.2010
(51) Int. Cl.: A61F 2/32, A61B 17/16, A61F 2/34, A61F 2/36

(54) **HIP JOINT DEVICE**
HÜFTGELENKVORRICHTUNG
DISPOSITIF D'ARTICULATION DE LA HANCHE

(30) Priority: 10.07.2009 SE 0900981; 10.07.2009 SE 0900957; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 10.07.2009 SE 0900958; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229752 P; 30.07.2009 US 229755 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P
(43) Date of publication of application: 16.05.2012
(73) Proprietor: Kirk Promotion LTD., 114 79 Stockholm (SE)
(72) Inventor: FORSELL, Peter, 6300 Zug (CH)
(86) International application number: PCT/SE2010/050831
(87) International publication number: WO 2011/005212

(56) References cited:
- EP-A2- 1 493 406
- EP-B1- 0 208 578
- WO-A2-02/09615
- WO-A2-03/011116
- US-A- 4 044 403
- US-A- 4 978 356
- US-A1- 2002 087 213
- US-B1- 6 290 727
- US-B1- 7 192 449
- US-B2- 6 986 792

## Description

### Technical field

The present invention relates generally to medical devices for implantation in a hip joint

### Background art

The hip joint is a synovial joint, joining the pelvis to the proximal portion of the femoral bone. Synovial joints are the most common types of joints in mammals, and are typical of nearly all limb joints. The contacting surfaces of said the pelvic, the acetabulum, and the contacting surface of the femoral bone, the caput femur, are smooth and rounded, and covered by articular cartilage. A synovial membrane, encapsulates the joint, forming a hip joint cavity, which contains synovial fluid. Outside the synovial membrane is a fibrous capsule and ligaments, forming an articular capsule.

There are both natural and pathological processes leading to deteriorated joint function. With age and wear, the articular cartilage becomes less effective as a shock absorber and a lubricated surface. Different degenerative joint diseases, such as arthritis, osteoartrithis, or osteoarthrosis, accelerate the deterioration.

Hip joint Osteoarthritis is a syndrome in which low-grade inflammation results in pain in the hip joints, caused by abnormal wearing of the Cartilage that acts as a cushion inside if the hip joint This abnormal wearing of the cartilage also results in a decrease of the joints lubricating fluid called Synovial fluid. Hip joint Osteoarthritis is estimated to affect 80% of all people over 65 years of age, in more or less serious forms.

The present treatment for hip osteoarthritis comprises NSAID drugs, local injections of Hyaluronic acid or Glucocorticoid to help lubricating the hip joint, and replacing parts of the hip joint with a prosthesis through hip joint surgery.

The replacing of parts of the hip joint is one of the most common surgeries to date performed at hundreds of thousands of patients in the world every year. The most common method comprises placing a metal prosthesis in Femur and a plastic bowl in Acetabulum. This operation is done through an incision in the hip and upper thigh and through Fascia lata and the lateral muscles of the thigh. To get access to the joint, the supporting Capsule attached to Femur and Ilium needs to be penetrated, making it difficult to get a fully functional joint after the surgery. Femur is then cut at the neck with a bone saw and the prosthesis is placed in femur either with bone cement or without. Acetabulum is slightly enlarged using an Acetabular reamer, and the plastic bowl is positioned using screws or bone cement.

The complications after hip joint surgery include dislocation of the hip joint and loosening of the prosthesis from its fixation in the femoral bone. The loosening and/or dislocation of the prosthesis could be induced by an abnormal strain being placed on the hip joint from e.g. the patient falling or making a rapid movement of the hip, or by a bodily macrophage reaction.

US Patent 7192449 (to McQeen et al.) shows a hip prosthesis having a shell, a liner and a retainer ring adapted to inhibit expansion of and decrease the size of the liner opening such that the prosthesis is fixated in the liner.

### Summary

A medical device according to claim 1, for implantation in a hip joint of a patient is provided. Preferred embodiments are defined in the dependent claims.

Please note that any embodiment or part of embodiment described herein or in the associated figures may be combined in any way.

### Brief description of drawings

The invention is now described, by way of example, with reference to the accompanying drawings, in which
Fig. 1 shows pelvis in a frontal view,
Fig. 2a shows pelvis in a lateral view,
Fig. 2b shows pelvis in a perspective view from below,
Fig. 2c shows the acetabulum, schematically,
Fig. 2d shows the acetabulum, schematically,
Fig. 3 shows the pelvis in a lateral view,
Fig. 4 shows the medical device according to one embodiment, in a perspective view,
Fig. 5 shows the medical device according to one embodiment, when fixated to the pelvic bone,
Fig. 6a shows pelvis in a frontal view, when medical device according to two embodiments have been implanted,
Fig. 6b shows pelvis in a frontal view, when medical device according to two further embodiments have been implanted,
Fig. 7 shows pelvis in a frontal view, when medical device according to two embodiments have been implanted,
Fig. 8 shows a medical device according to one embodiment, when placed on a prosthetic caput femur fixated in the femoral bone.
Fig. 9a shows pelvis in a lateral view, when the medical device is implanted,
Fig. 9b shows pelvis in a lateral view, when the medical device is implanted,
Fig. 10 shows pelvis in a lateral view, when the medical device is implanted,
Fig. 11 shows pelvis in a lateral view, when the medical device is implanted,
Fig. 12 shows pelvis in a lateral view, when the medical device is implanted,
Fig. 13 shows the medical device according to yet another embodiment,
Fig. 14a shows the medical device according to one embodiment with two extending portions,
Fig. 14b shows the medical device according to one embodiment with three extending portions,
Fig. 14c shows the medical device according to one embodiment with three extending portions,
Fig. 14d shows the medical device according to one embodiment with four extending portions,
Fig. 14e shows the medical device according to one embodiment with two extending portions,
Fig. 14f shows the medical device according to one embodiment with three extending portions,
Fig. 15a shows the hip joint in section when a medical device is implanted, in its first state,
Fig. 15b shows the hip joint in section when a medical device is implanted, in its second state,
Fig. 16 shows the hip joint in section when a medical device is implanted, in its first state,
Fig. 17 shows the hip joint in section when a medical device is implanted, in its second state,
Fig. 18 shows the medical device in section, when in its first state,
Fig. 19 shows the hip joint in section when a medical device is implanted, in its first state,
Fig. 20 shows the hip joint in section when a medical device is implanted, in its second state,
Fig. 21 shows the hip jointin section when a medical device is implanted, in its first state,
Fig. 22 shows the hip joint in section when a medical device is implanted, in its second state,
Fig. 23a shows the hip jointin section when a medical device is implanted, in its first state,
Fig. 23b shows a medical device in section when in its first state,
Fig. 24a shows the hip joint in section when a medical device is implanted, in its second state,
Fig. 24b shows a medical device in section when in its second state,
Fig. 25 shows the hip jointin section when a medical device is implanted, in its first state,
Fig. 26 shows the hip joint in section when a medical device is implanted, in its second state,
Fig. 27 shows the hip joint in section when a medical device is implanted, in its first state,
Fig. 28 shows the hip joint in section when a medical device is implanted, in its second state,
Fig. 29 shows the hip joint in section when a medical device is implanted, in its first state,
Fig. 30 shows the hip joint in section when a medical device is implanted, in its second state,
Fig. 31 shows the hip joint in section when a medical device is implanted, in its first state,
Fig. 32 shows the hip joint in section when a medical device is implanted, in its second state,
Fig. 33 shows the hip joint in section when a medical device is implanted, in its first state,
Fig. 34 shows the hip joint in section when a medical device is implanted, in its first state,
Fig. 35 shows the hip joint in section when a medical device is implanted, in its second state.

### Detailed Description

The hip joint is a synovial ball and socket joint which permits a large motion range for allowing a plurality of different movements of the lower limb. From a neutral position the following movements of the hip joint are normally possible: lateral or external rotation, 30° with the hip extended, 50° with the hip flexed, medial or internal rotation 40°, extension or retroversion 20°, flexion or anteversion 140°, abduction 50° with hip extended, 80° with hip flexed, adduction 30° with hip extended, 20° with hip flexed.

When replacing the natural hip joint with a prosthetic hip joint, the depth of the prosthetic acetabulum will affect the motion range, the deeper the acetabulum bowl is made the more restrictive it is to the motion range. A deeper bowl has the advantage of reducing the risk of hip joint luxation, the risk of which is a major drawback with prosthetic hips of today.

The anatomy of the hip joint and its surroundings is further disclosed in: Marieb etal., Human Anatomy, 2003, Benjamin Cummings, San Fhancisco, pages 195 - 202 and in Moore etal., Clinically oriented anatomy, 1999, Lippincott, Williams & Wilkins, Baltimore, pages 501 - 653, both hereby incorporated by reference.

Functional hip movements are to be understood as movements of the hip that at least partly correspond to the natural movements of the hip. On some occasions the natural movements of the hip joint might be some what limited or altered after hip joint surgery, which makes the functional hip movements of a hip joint with prosthetic surfaces somewhat different than the functional hip movements of a natural hip joint

Everyday activities is to be understood as activities which are not connected to any extreme movements, such that some physical sports require. For example, everyday activities comprise: walking, sitting, cycling etc.

The functional position of an implantable medical device or prosthesis is the position in which the hip joint can perform functional hip movements. The final position is to be understood as a functional position in which the medical device needs no further position change to function.

Elastic deformation is when a material deforms under stress (e.g. external forces), but returns to its original shape when the stress is removed. A more elastic material is to be understood as a material having a lower modulus of elasticity. The elastic modulus of an object is defined as the slope of its stress-strain curve in the elastic deformation region The elastic modulus is calculated as stress / strain, where stress is the force causing the deformation, divided by the area to which the force is applied; and strain is the ratio of the change caused by the stress.

Elasticity is to be understood as a materials ability to deform in an elastic way.

Stiffness is to be understood as the resistance of an elastic body to deformation by an applied force.

Biocompatible material is to be understood as being a material with low level of immune response. Biocompatible materials are sometimes also referred to as biomaterials. Analogous is biocompatible metals a biocompatible metal with low immune response such as titanium or tantalum. The biocompatible metal could also be a biocompatible alloy comprising at least one biocompatible metal.

Form fitting is to be understood as an element having a part or section which is adapted to enable a mechanical connection of said element to atleastone other element using said part or section. Form fitted structure is a structure of an element which enables form fitting.

In the following a detailed description of embodiments of the present invention will be given In the drawing figures, like reference numerals designate identical or corresponding elements throughout the several figures. It will be appreciated that these figures are for illustration only and are not in any way restricting the scope of the invention. Thus, any references to direction, such as "up" or "down", are only referring to the directions shown in the figures. Also, any dimensions etc. shown in the figures are for illustration purposes.

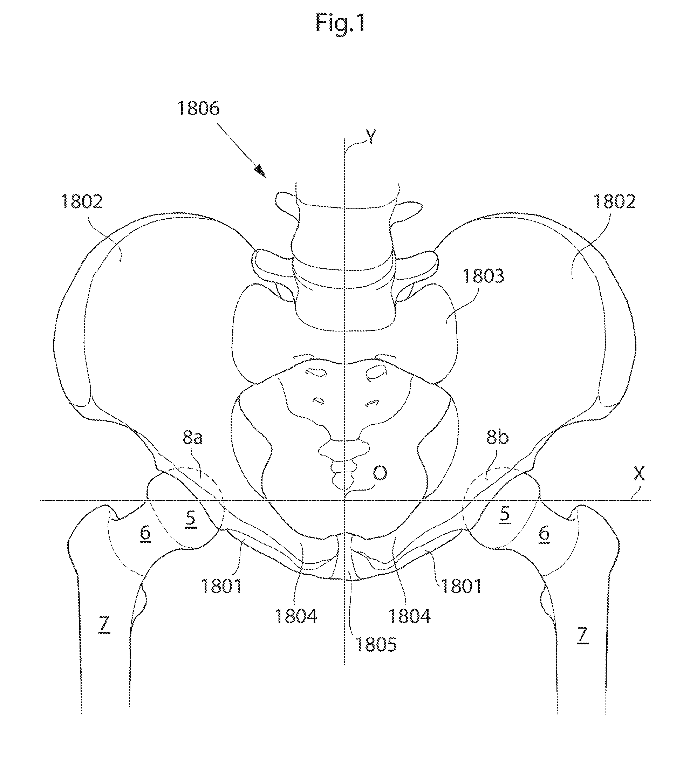

Fig. 1 shows the pelvis in a frontal view. Pelvis comprises the right and left hip bone making up the pelvic bone, in turn comprising the Sacrum 1803, Ilium 1802, Pubis 1804 and Ischium 1801. The hip joint houses the right and left acetabulum 8a,b placed laterally and distally in the pelvis. The acetabulum 8a,b being a spherically shaped cavity in the hip bones making up one of the parts of the hip joint, the acetabulum 8a,b being adapted to house the caput femur 5, being the proximal portion of the femoral bone 7 having a spherical contacting surface adapted to be placed in the acetabulum 8a,b and thus creating the operable hip joint The pelvis has a lateral-medial axis X extending substantially from the bottom of the left acetabulum 8a to the bottom of the right acetabulum 8b, the pelvis further having a proximal-distal Y axis extending perpendicular to said lateral-medial axis, centrally and substantially along the length of the patient, passing the dorsal portions of the pubic symphysis 1805 and substantially following the spinal cord 1806, intersecting the lateral-medial axis X.

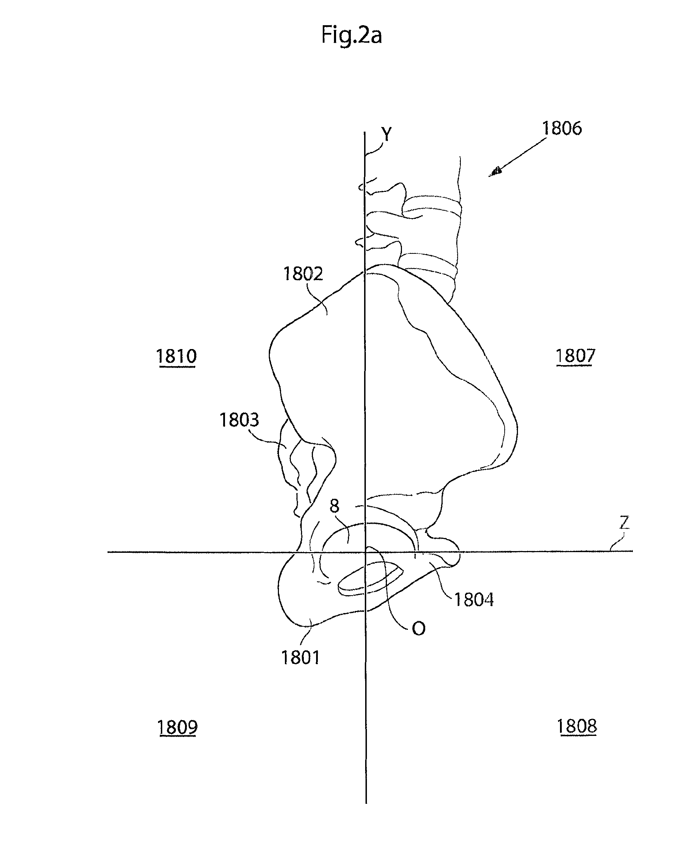

Fig.2a shows the pelvis in a lateral view, thus displaying the posterior side of Ilimu 1802, the anterior side of Ichum 1801, the anterior side of Pubis 1804, and Sacrum 1803 in a strict lateral view. The pelvis has furthermore a frontal-dorsal axis Z being perpendicular to the proximal-distal axis Y and the lateral-medial axis shown in fig. 1, and intersecting them both creating a common origin O for the three axis X,Y,Z. The frontal-dorsal axis Z and the proximal-distal axis Y thus being oriented such that a first plane PZ, extending from the frontal-dorsal axis Z, and a second plane PY, extending from the proximal-distal axis Y, penetrates through the acetabulum 8, thus dividing the acetabulum 8 into 4 sections 1807, 1808, 1809 and 1810.

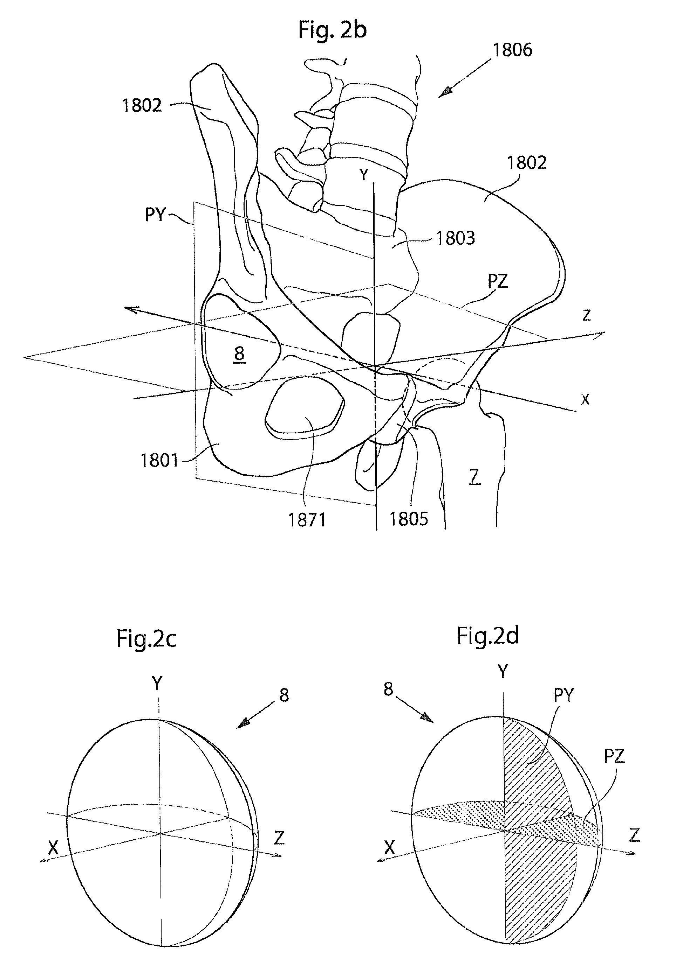

Fig. 2b shows the pelvis in a perspective view from below displaying the lateral-medial axis X passing through the center of the right and left acetabulum 8. The lateral-medial axis X is perpendicular to the frontal-dorsal axis Z which also is perpendicular to the proximal-distal axis Y. A first plane PY extends from the proximal-distal axis Y, thus dividing the acetabulum in half, centrally. A second plane PZ extends from the dorsal-frontal axis Z, thus dividing the acetabulum in half, centrally and perpendicularly to the first plane PY.

Fig. 2c and 2d shows the acetabulum 8 schematically, and how the planes PY, PZ devides the acetabulum in to quarters with axis X,Y,Z parallel to the X,Y,Z axis disclosed previously. Fig 2b further discloses foramen obturatum 1871.

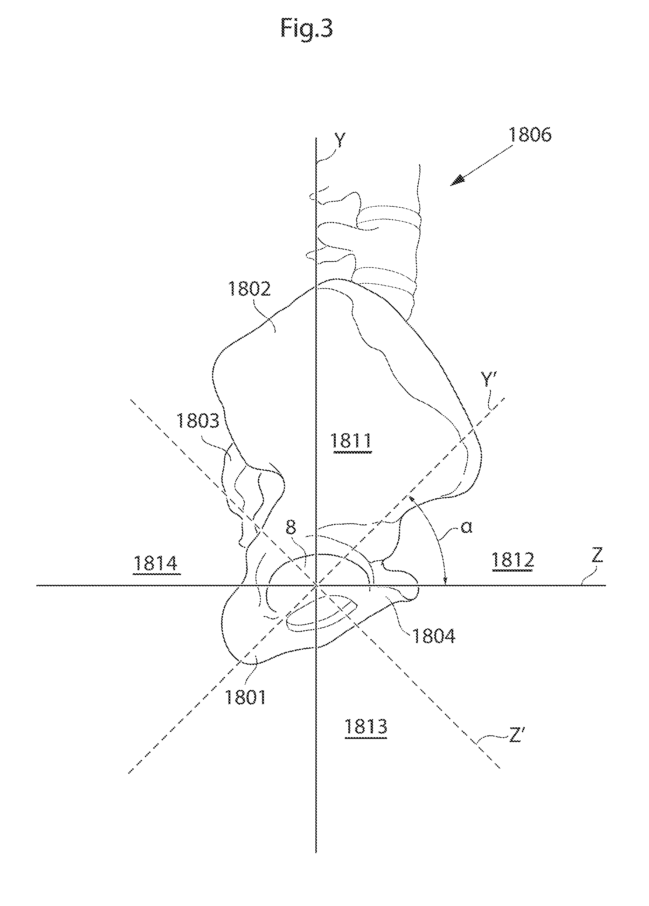

Fig. 3 shows a lateral view of the pelvis and further displaying two perpendicular axis Y'and Z' being two axis with the same origin as the axis Y and Z, but rotated clockwise at an angle α being 45°. The two axis Y' and Z' thus dividing the acetabulum 8 into quadrants 1811, 1812, 1813 and 1814. The quadrants being a proximal quadrant 1811, a frontal quadrant 1812, a distal quadrant 1813 and a dorsal quadrant 1814.

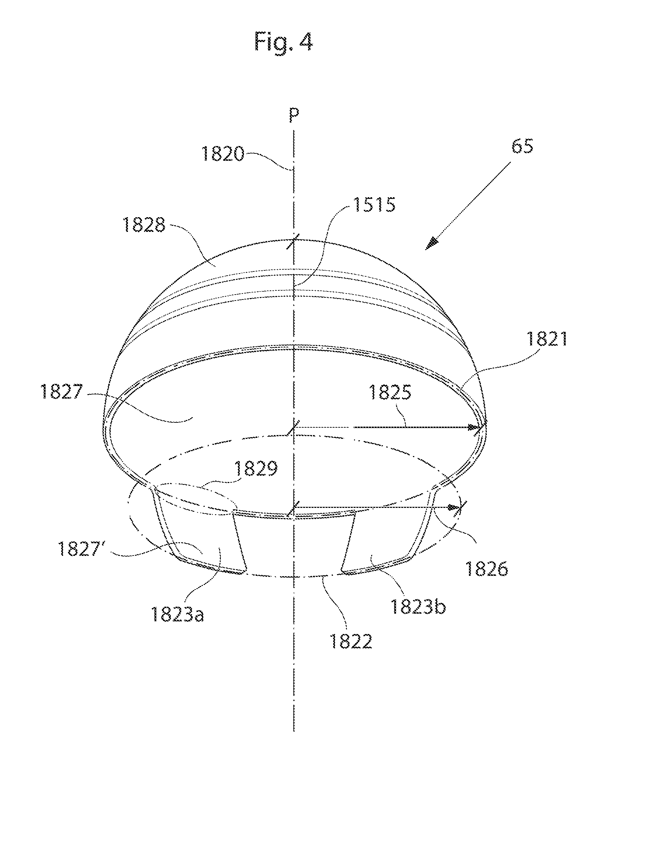

Fig. 4 shows a medical device for implantation in a hip joint of a patient. The medical device is adapted to be fixated to the pelvic bone of the patient for example by means of an adhesive, such as bone cement, or mechanical fixating members, such as orthopedic screws. The medical device comprises an inner 1 827 and an outer 1 828 surface. A contacting portion of the inner surface 1827 is spherical and faces the center of the hip joint, when the medical device is implanted. The inside of the medical device is adapted to receive a caput femur or a prosthetic replacement therefor having a spherical portion, and the spherical contacting portion of the inner surface 1827 is adapted to be in contact with a spherical portion of the outer surface of the caput femur or a prosthetic replacement therefor. The medical device, according to the embodiment shown in fig. 4 comprises two extending portions 1823a,b, extending the contacting portion of the inner surface 1827' such that the extending portions 1823a,b clasps the spherical portion of caput femur or a prosthetic replacement therefor, for restraining the spherical portion in the medical device. The medical device is adapted to receive the caput femur or a prosthetic replacement therefor, having a collum femur or prosthetic collum femur fixated to the spherical portion of the caput femur or prosthetic replacement therefor. The inner surface 1827 comprises an equator line 1821, being the largest circular circumference of the inner surface. The two extending portions passes beyond the equator line 1821, such that and end portion 1829 of the contacting portion, here being of the extending portion 1823b of the inner surface 1827, forms a circular extension line 1822 placed distal to the equator line 1821, when the medical device is implanted, and having a smaller circumference than the equator line 1821; thus a distance 1826 between a center axis P of the medical device and the extension line 1822 is shorter than a distance 1825 between the center axis P and the equator line 1821.

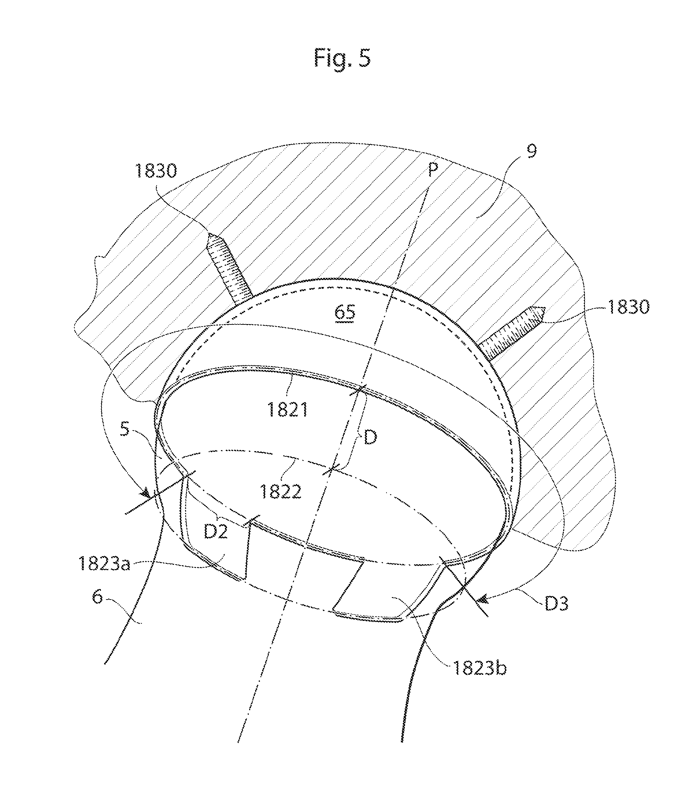

Fig. 5 shows the medical device described with reference to fig. 4 when implanted. According to this embodiment the medical device is adapted to be fixated using orthopedic screws 1830, mechanically fixating the medical device to the pelvic bone 9, by the medical device comprising holes through which the screws 1830 are placed. In fig. 5 the contacting portion of the inner surface 1827 has been placed in contact with the spherical portion of a prosthetic caput femur 5 being fixated to a prosthetic collum femur 6, the prosthetic caput 5 and collum 6 femur replacing the proximal portion of the femoral bone. The two extending portions 1823a and 1823b extending the contacting portion of the inner surface and clasps the spherical portion of the prosthetic caput femur 5, for restraining the spherical portion in the medical device. The inner surface comprising the equator line 1821, and the extending portions 1823a,b passing beyond the equator line 1821 and comprising the more distal extension line 1822 having a smaller circumference than the equator line 1821. The more distal extension line 1822 being placed at a distance D1 from the equator line 1821. According to this embodiment the extension line 1822 is parallel to the equator line 1821, however this is not necessarily so in other embodiments.

The extension portion 1823a according to the embodiment shown in fig. 5 extends longitudinally along the equator line, a distance D2. Along another portion of the equator line, a distance D3, there are no extending portion, which enables the collum femur 6 to enter the space between the first and second extending portions 1823a,b which creates a larger movement range of the hip joint

The extending portions thus extending discontinuously along the equator line 1821, such that a portion of the collum femur 6 can be placed between the extension line 1822 and the equator line 1821.

The extending portion, according to any of the embodiments, adapted to clasp the caput femur or prosthetic replacement therefor for restraining the caput femur or prosthetic replacement therefor in the medical device, could further be adapted to release the caput femur or prosthetic replacement therefor when a large enough strain is placed on the joint This feature enables the caput femur or prosthetic replacement therefor to be fixedly attached in the medical device in normal use and be released from the medical device e.g. in case of an accident, thus reducing the risk of damaging the bodily structures, such as the femoral bone, or the fixations between bodily structures and prosthetic parts, such as the fixation between the femoral bone and a prosthetic stem to which the prosthetic collum and caput femur is fixated.

According to one embodiment the extending elements, as for example disclosed with reference to figs. 1 - 5, are placed such that the extending elements restricts the motion range minimally, or in ways which are not limiting the motion range used in everyday life. The hip joint is a synovial ball and socket joint which permits a large motion range for allowing a plurality of different movements of the lower limb. From a neutral position the following movements of the hip joint are normally possible: lateral or external rotation, 30° with the hip extended, 50° with the hip flexed, medial or internal rotation 40°, extension or retroversion 20°, flexion or anteversion 140°, abduction 50° with hip extended, 80° with hip flexed, adduction 30° with hip extended, 20° with hip flexed. In the movement ranges of abduction and adduction the depth of the acetabulumbowl and thus the extending portions does not restrict the motion range in a critical way since the motion range of the normal hip is restricted in these movements, in normally agile persons, by the muscles, tenors and ligaments surrounding the hip joint

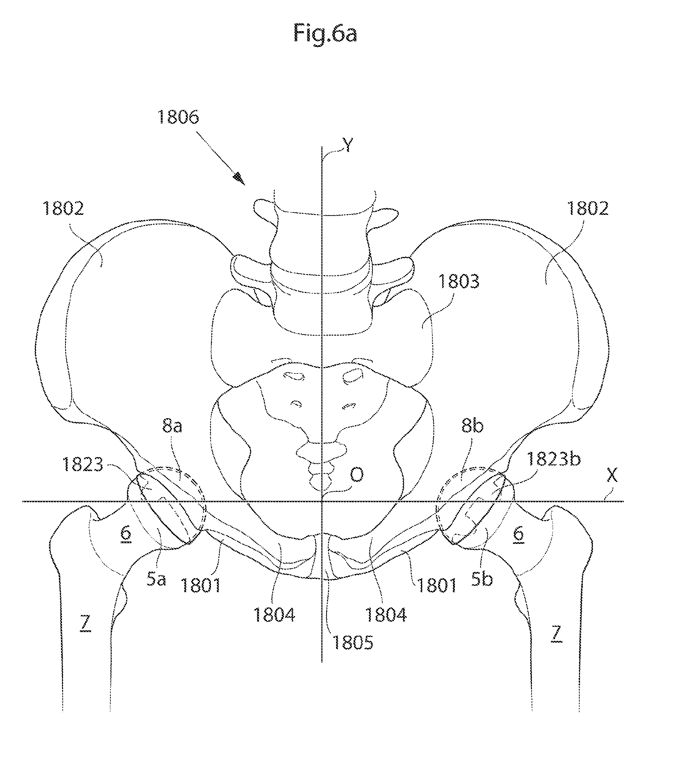

Fig. 6a shows a frontal view of pubis and the proximal portions of the femoral bones 7 when two embodiments of the medical device has been implanted in the hip joint The medical device shown placed on the right caput femur 5a and placed in the right acetabulum 8a comprises one extending portion 1823, here placed dorsal to the proximal-distal axis Y, thus only partially limiting abduction in far excess of 50°. According to the embodiment shown, the extending portion 1823 extends longitudinally along the equator line 1821 about 1/ 10 of the length of the equator line 1821, however in other embodiments the extending portion 1823 extends along as much as half of the length of the equator line 1821, and in other embodiments the extending portion 1823 extends as little as about 1/ 30 of the length of the equator line 1821. The medical device shown placed on the left caput femur 5b and placed in the left acetabulum 8b comprises two extending portions 1823a,b, both being placed dorsal to the proximal-distal axis Y, when implanted, thus limiting the motion range of the hip joint in a non restrictive way, in relation to everyday activities. In both the right and left embodiment the extending portions 1823 extends discontinuously along the equator line 1821 thus enabling the collum femur 6 to partially be placed between the equator line and the extension line, and in the left embodiment, placed between the extending portions 1823a,b thus entering the cavity between the extending portions 1823a,b.

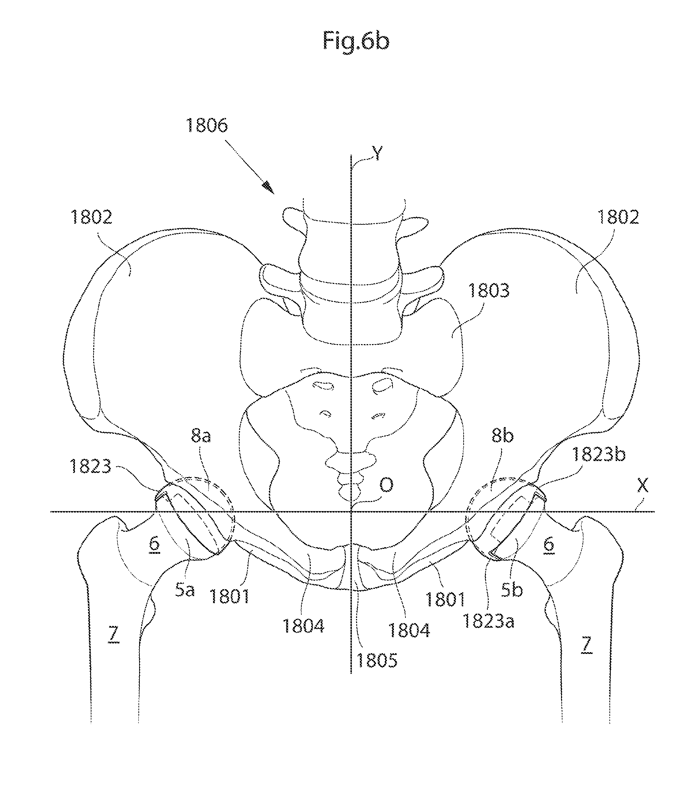

Fig. 6b shows a frontal view of pubis and the proximal portions of the femoral bones 7 when two further embodiments of the medical device have been implanted in the hip joint The medical device shown placed on the right caput femur 5a and placed in the right acetabulum 8a comprises one extending portion 1823, here placed in the proximal quadrant, which is further disclosed with reference to fig. 3, thus limiting the motion range of the hip joint in a non restrictive way, in relation to everyday activities. According to the embodiment shown, the extending portion 1823 extends longitudinally along the equator line 1821 about 1/ 10 of the length of the equator line 1821, however in other embodiments the extending portion 1823 extends along as much as half of the length of the equator line 1821, and in other embodiments the extending portion 1823 extends as little as about 1/ 30 of the length of the equator line 1821. The medical device shown placed on the left caput femur 5b and placed in the left acetabulum 8b comprises two extending portions 1823a,b, being placed in the proximal and distal quadrants, thus limiting the motion range of the hip joint in a non restrictive way, in relation to everyday activities.

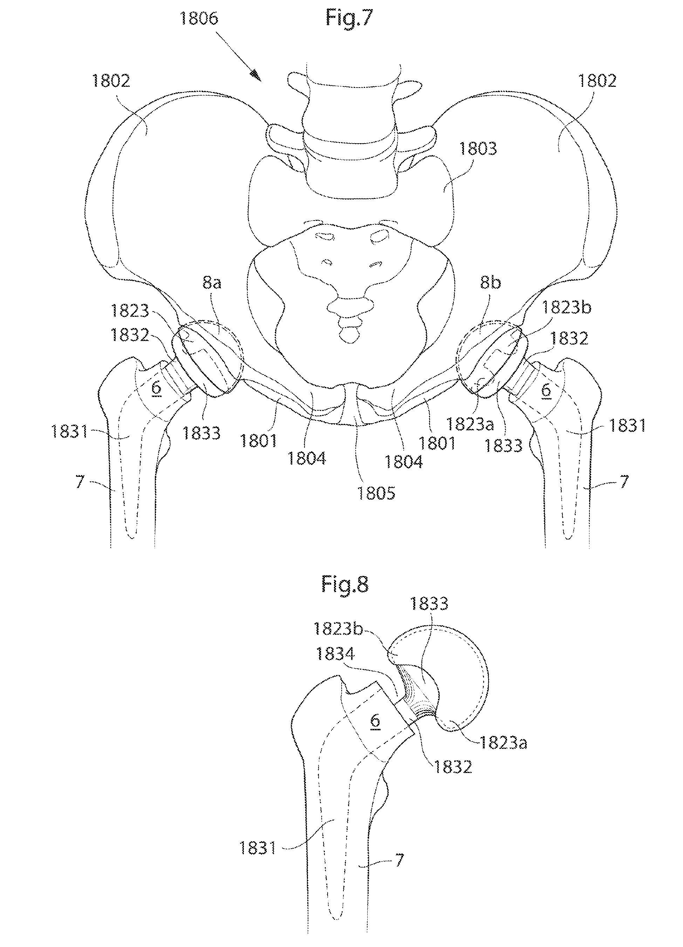

Fig. 7 shows the pelvis and the proximal portions of the femoral bones 7 including the embodiment of fig. 6a, with the difference that the natural caput femur and a portion of the natural collum femur has been replaced by a prosthetic caput femur 1833 and a prosthetic collum femur 1832. The prosthesis further comprises a prosthetic stem 1831 adapted to be placed inside and fixated to the femoral bone, either using bone cement or by the surface of the stem being adapted to facilitate the growth-in of bone, thus fixating the stem The prosthetic collum femur 1832 could be coordinated with the extending portions 1823 of the medical device for further improving the motion range of the hip joint, or not limiting the natural motion range of the hip joint

Fig. 8 shows the medical device according to an embodiment in which the medical device comprises two extending portions 1823a,b. The medical device is placed on a prosthetic collum femur 1832, to which a prosthetic caput femur 1833 is attached. The prosthesis further comprises a stem 1831 which is adapted to be fixated inside of the femoral bone 7. The prosthetic collum femur 1832 is here adapted to further improve the motion range of the hip joint, or not limiting the natural mo tio n range of the hip joint, by the prosthetic collum femur 1832 comprising a cavity 1834 in which the extending portions 1823 can be placed.

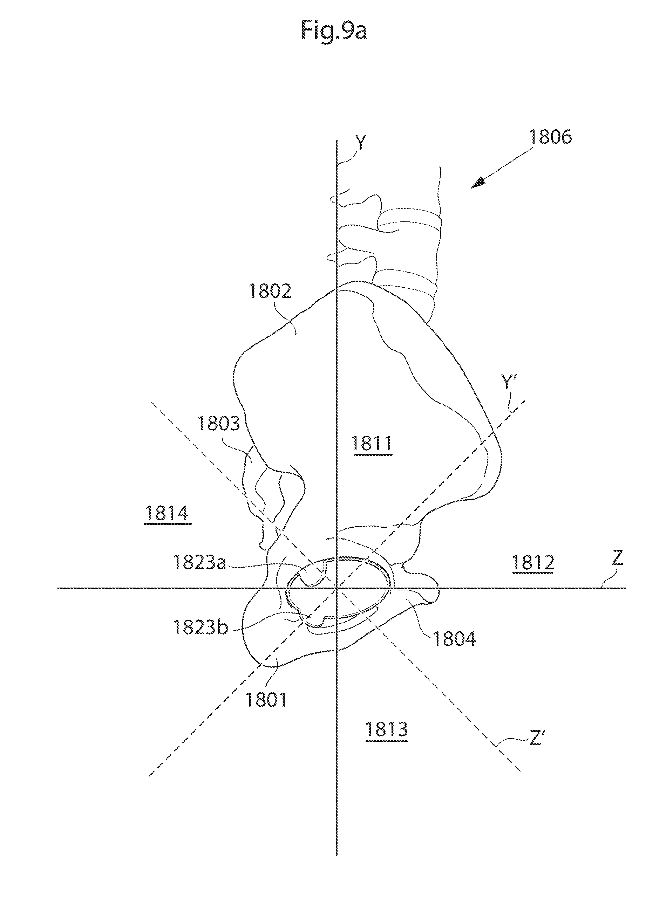

Fig. 9a shows the pelvis in a lateral view, the medical device comprises two extending portions 1823a,b, both extending longitudinally along the equator line (as disclosed in for example fig. 5) dorsal to the proximal-distal axis Y and being adapted to clasp the caputfemurora prosthetic replacement therefor. The extending portions 1823a,b extending dorsal to the proximal-distal axis Y and thus reducing the limiting effect that the extending portions 1823a,b, have on the motion range of the hip joint According to the embodiment shown in fig. 9a the extending portion 1823a placed proximally in the acetabulum extends longitudinally a distance of about ¼ of the length of the equator line, and the extending portion 1823b placed distally in the acetabulum extends longitudinally a distance of about 1/ 10 of the length of the equator line, however it is equally conceivable that this relationship is the other way around, or that any of the extending portions longitudinally extends a distance of as much as half of the length of the equator line, thus extending the entire distance of the equator line being dorsal to the proximal-distal axis, or that any of the extending portions 1823a,b extends a distance being as little as 1/ 30 of the distance of the equator line.

According to the embodiment shown in fig. 9a, the first extending portion 1823a extends in distal-lateral direction from the acetabulum, and the second extending portion 1823b extends medially towards foramen obturatum.

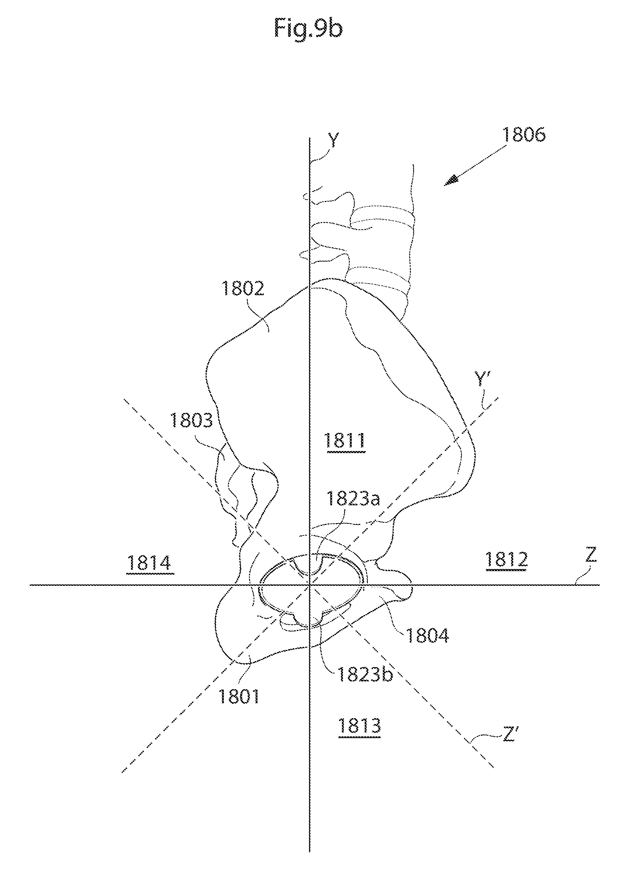

Fig. 9b shows the pelvis in a lateral view, the medical not falling under the invention device comprises two extending portions 1823a,b, the two extending portions 1823a,b extends in the proximal quadrant 1811 and the distal quadrant 1813, respectively.

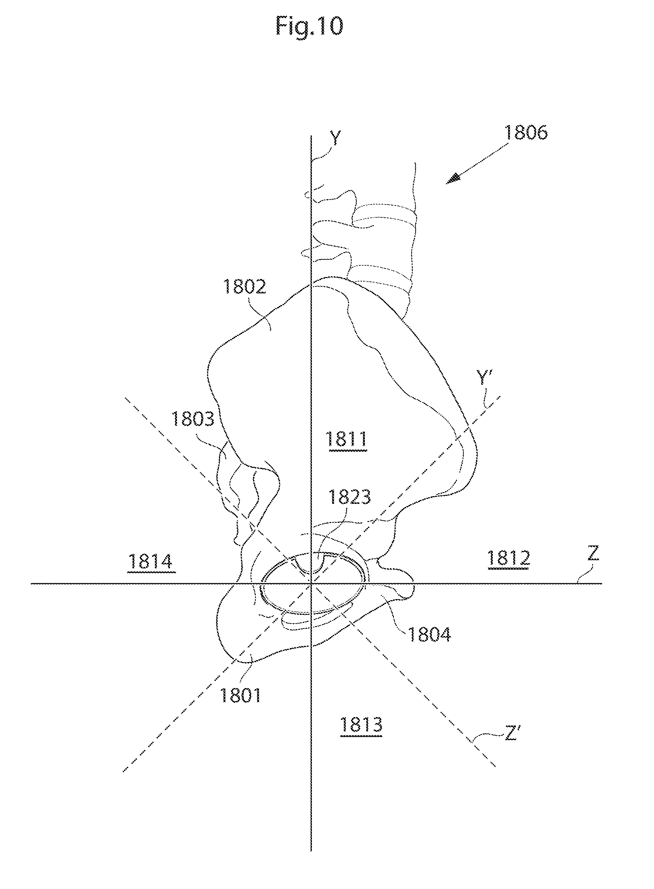

There are multiple ways in which the extending portions 1823 can be adapted to reduce the effects that the extensions have on the motion range of the hip joint Fig. 10 shows the pelvis in a lateral view, the medical device not falling under the invention comprises one extending portion 1823 extending and being adapted to clasp the caput femur, or a prosthetic replacement therefor. The extending portion 1823 extends longitudinally along the equator line within the proximal quadrant 1811, which is further disclosed with reference to fig. 3. According to the embodiment shown in fig. 10, the extending portion 1823 extends in distal-lateral direction from the acetabulum.

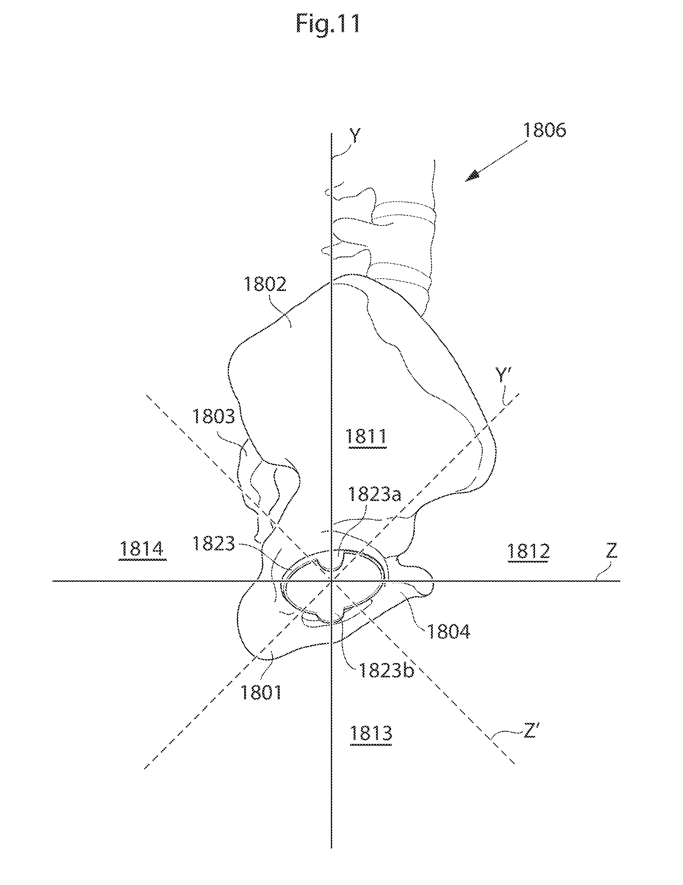

Fig. 11 shows the pelvis in a lateral view, the medical device not falling under the invention comprises a continuously extending portion 1823 with two extending portions 1823a and 1823b extending further in relation to the average extension of the extending portion. The entire extending portion is placed in the proximal, distal and dorsal quadrants and the extending portions 1823a,b extending further than the average extension of the extending portion 1823 extends in the proximal and distal quadrant

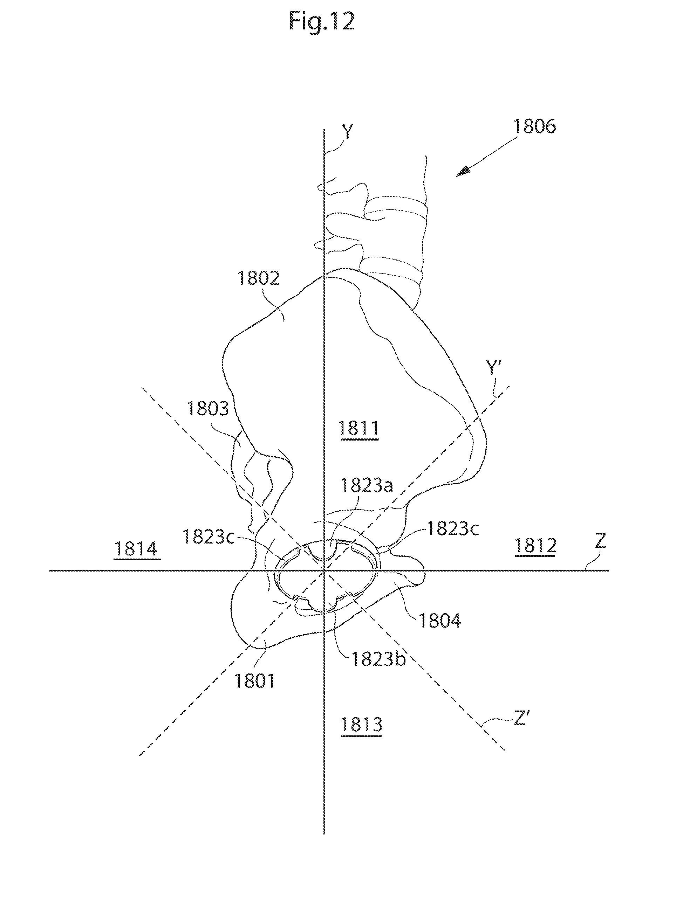

Fig. 12 shows the pelvis in a lateral view, the medical device not falling under the invention comprises four extending portions 1823a,b,c,d, wherein the first 1823a and second 1823b extending portions extends in the proximal and distal quadrant, respectively, thus the first extending portion 1823a extending in distal-lateral direction from the acetabulum, and the second extending portion 1823b extending medially towards foramen obturatum. The third extending portion 1823c extending in the frontal quadrant 1812, out from the acetabulum in dorsal direction, extends less than the first and second extending portion, since extending portions 1823c in the frontal quadrant is more limiting to the normal motion range of the hip joint The fourth extending portion 1823d extends in the dorsal quadrant in accordance with the third extending portion 1823c do not extend as far as the first and second extending portions.

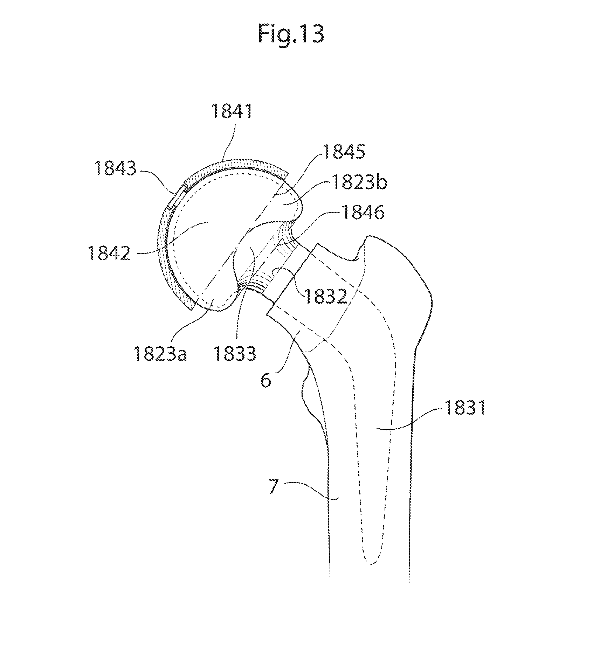

Fig. 13 shows an alternative embodiment of the medical device not falling under the invention. In the alternative embodiment the medical device comprises a first part 1841 adapted to be fixated to the pelvic bone of the patient The first part comprises an inner contacting surface adapted to be in movable connection with an outer contacting surface of a second part 1842. The second part 1842 is rotatably fixated to the first part 1841 by a rotatable connecting member 1843. An outer contacting surface of a prosthetic caput femur 1833 is adapted to be placed in contact with the inner surface of the second part 1842 and be movable in multiple directions, thus replicating the natural ball and socketjointofthe hip. The second part 1842 comprises two extending portions 1823a,b extending beyond the equator line 1845 of the second part 1842. The extending portions 1823a,b extends longitudinally discontinuously along the equator line, thus creating an area between the extending portions, in which area a portion of the prosthetic collum femur can be placed, thus being placed partially between the equator line 1845 and the extension line 1846. The construction shown in fig. 13 enables the second part 1842 to rotate if the collum femur 1832 engages the extending portions 1823a,b, which are sloped for this purpose. This way the second part 1842 are always placed such that the collum femur 1832 can be placed partially between equator line 1845 and the extension line 1846, which creates an optimal range of movement whilst the second partclasps the prosthetic caput femur 1833, and thus restricting the caput femur 1833 in the second part 1842 of the medical device.

According to the embodiment shown the caput and collum femur is a prosthetic caput 1833 and collum 1832 femur, comprising a prosthetic stem 1831 adapted to be fixated in the femoral bone 7, however, in other embodiments, it is equally conceivable that the natural caput femur is resurfaced and placed in the second part 1842.

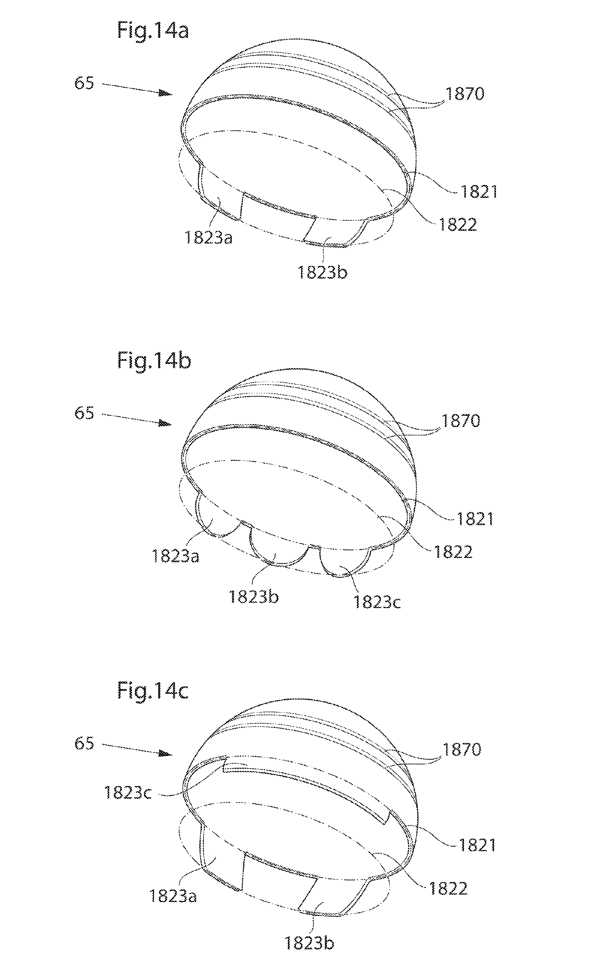

Fig. 14a shows the medical device 65 in a perspective view from below according to one embodiment In this embodiment the medical device comprises two extending portions 1823a, b. The medical device 65 is according to this embodiment adapted to be fixated to the pelvic bone by means of an adhesive which is adapted to be placed in connection with the adhesive recesses 1870 of the outer surface of the medical device 65.

Fig. 14b shows a medical device similar to the medical device disclosed with reference to fig. 14a, but with the difference that it comprises three equally extending portions 1823a,b,c.

Fig. 14c shows a medical device not falling under the invention similar to the medical device disclosed with reference to fig. 14a, but with the difference that it comprises two equally extending portions 1823a,b and one less extending portion 1823c.

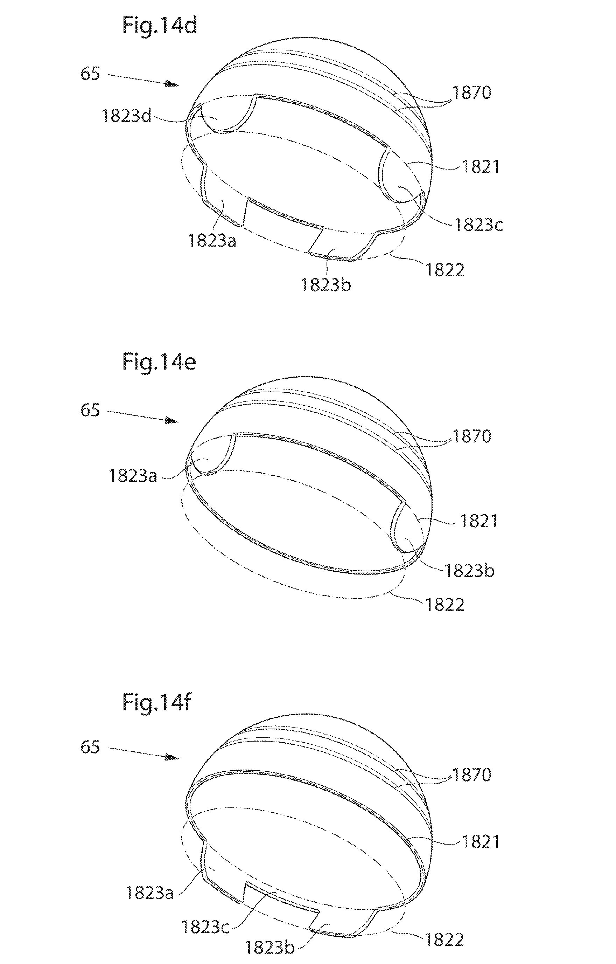

Fig. 14d shows a medical device not falling under the invention similar to the medical device disclosed with reference to fig. 14a, but with the difference that it comprises four equally extending portions 1823a,b,c,d.

Fig. 14b shows a medical device similar to the medical device disclosed with reference to fig. 14a, but with the difference that the two extending portions are placed further from each other, and thus being adapted to be placed in the proximal and distal quadrant, when implanted.

Fig. 14f shows a medical device similar to the medical device disclosed with reference to fig. 14a, but further comprising a less extending portion 1823c placed between the first and second extending portions 1823a,b.

The extending portions of the medical device which have been described could be made from an elastic material, enabling the extending portions to pass onto the caput femur.

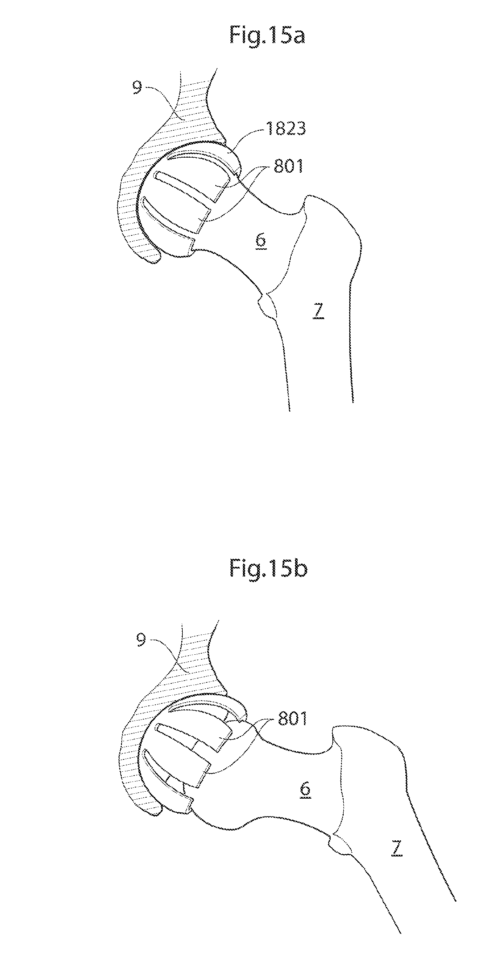

Fig. 15a shows the medical device in an embodiment in which the medical device is fixated to the pelvic bone 9. The medical device comprises extending portions which in turn serves as releasing members 801 adapted, in a first state, to hold the caput femur in the medical device and in a second state release the caput femur 5 from the medical device. The releasing member 801 is adapted to change from the first state to the second state when a pre-determined strain is placed on the releasing member 801.

The strain could be caused by an abnormal movement of the hip joint, e.g. as the result of the patient falling. According to the embodiment shown in fig. 14 the releasing member 801 comprises an elastic portion comprising elastic material, in the embodiment shown being the entire releasing member 801. The releasing member is adapted to non-invasively be able to change from the first state to the second state and from the second state to the first state, when a pre-determined strain is placed on the releasing member 801 i.e.

Fig. 15b shows the hip joint in section when the releasing member 801 is in its second state, wherein the releasing member 801 is adapted to release the caput femur 5 from the medical device placed in the pelvic bone 9. The releasing member 801 has changed from the first state to the second state because a pre-determined strain has been placed on the releasing member 801.

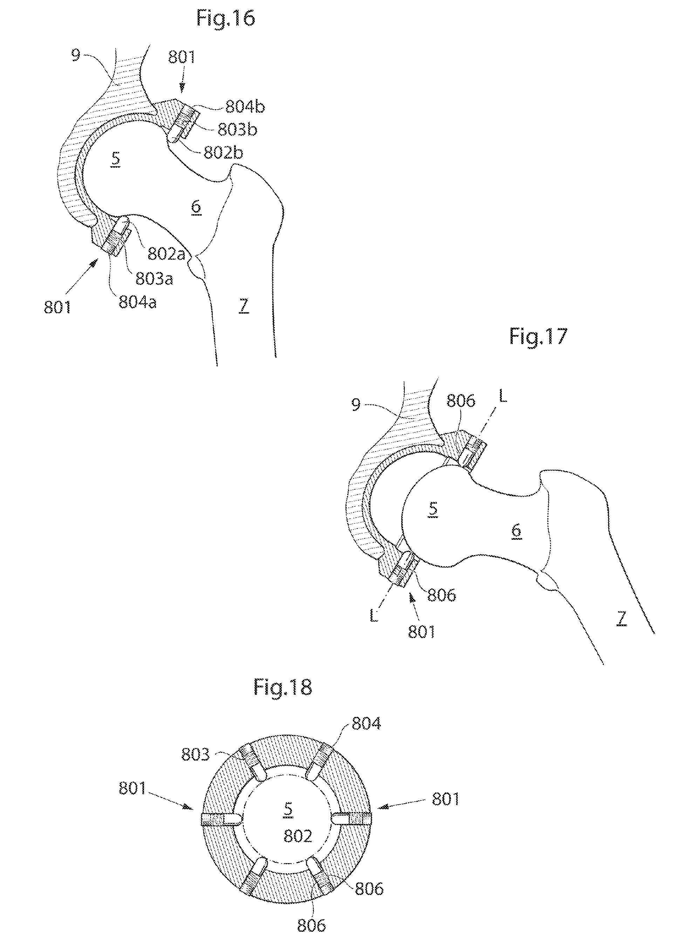

Fig. 16 shows the medical device according to an embodiment where the medical device comprises holding members 802a,b, adapted to slide against the caput femur 5, or a prosthetic replacement therefore. The holding members are adapted to, in a first state, hold the caput femur 5, or a prosthetic replacement therefore, in a second state the releasing member 801 is adapted to release the caput femur 5, or a prosthetic replacement therefore, from the medical device placed in the pelvic bone 9. The holding members 802a,b are spring loaded through a spring 803a,b being placed between a calibration member, being a calibration screw 804a,b, and the holding members 802a,b. The force exerted on the holding members 802a,b from the spring 803a,b is adapted to hold the caputfemur 5, or a prosthetic replacement therefore, in the medical device in normal, functional hip joint movements, but release the caputfemur5, ora prosthetic replacement therefore, from the medical device when a pre-determined strain is placed on the releasing member which could be caused by an abnormal movement of the hip joint, e.g. as the result of the patient falling. The calibration screws 804a,b enables the pre-determination of the strain which will cause the holding members 802a,b to change from being in a first state to being in a second state.

Fig 17 shows the releasing members in their second state, when a pre-determined strain has been exceeded, preferably being caused by an abnormal movement of the hip joint, e.g. as the result of the patient falling. The holding members 802a,b are retracted into sleeves 806 of the medical device, thereby compressing the springs 803a,b. The retraction of the holding members 802a,b causes the caputfemur 5, or a prosthetic replacement therefore, to be dislocated/ luxated from its position in the medical device, which, when large strain is placed on the hip joint and femoral bone 7, reduces the risk of the patient fracturing the femoral bone 7 or the pelvic bone 9. The holding members 802a,b are adapted to non-invasively be able to change from the first state to the second state and from the second state to the first state, when a pre-determined strain is placed on the holding members 802a,b.

Fig. 18 shows the medical device in section, with the holding members 802, placed in sleeves 806 evenly distributed along the cross-section of the medical device, holding the caput femur 5, or a prosthetic replacement therefore, in position in the medical device.

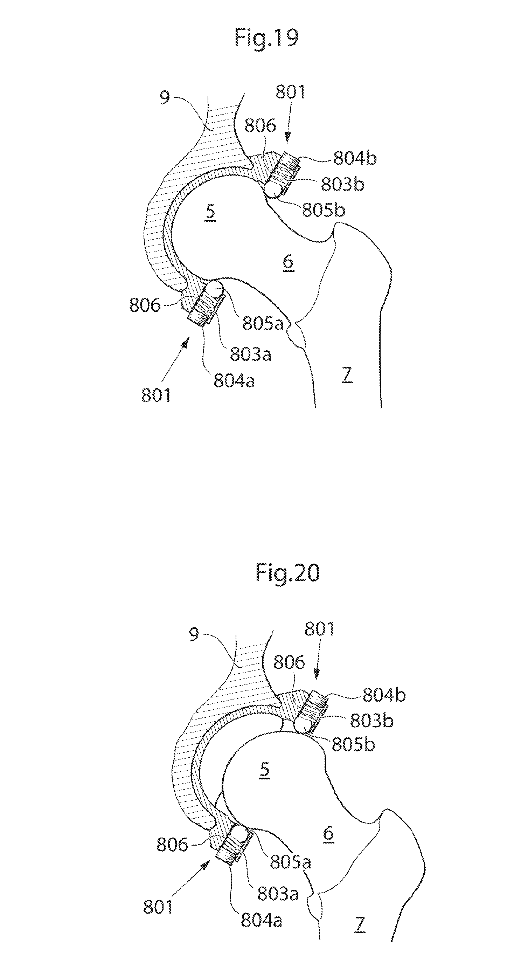

Fig. 19 shows an alternative embodiment of the principle shown in figs 16-18, wherein the holding members 802a,b, comprises ball shaped members 805a,b in contact with the caputfemur5, or a prosthetic replacement therefore, and being adapted to roll against the caputfemur5, or a prosthetic replacement therefore, holding the caputfemur 5, or a prosthetic replacement therefore, in place in the medical device, by the holding members 802a,b exerting force on the caputfemur 5, or a prosthetic replacement therefore, through the contact with the springs 803a,b supported by the calibration screws 804a,b.

Fig 20 shows the releasing members in their second state, when a pre-determined strain has been exceeded, preferably being caused by an abnormal movement of the hip joint, e.g. as the result of the patient falling. The holding members 802a,b, comprising the ball shaped members 805a,b, are retracted into sleeves 806 of the Medical device, thereby compressing the springs 803a,b. The retraction of the holding members 802a,b causes the caputfemur5, or a prosthetic replacement therefore, to be dislocated/ luxated from its position in the medical device, which, when large strain is placed on the hip joint and femoral bone 7, reduces the risk of the patient fracturing the femoral bone 7 or the pelvic bone 9. The holding members 802a,b are adapted to non-invasively be able to change from the first state to the second state and from the second state to the first state, when a pre-determined strain is placed on the holding members 802a,b, which enables the caput femur 5, or a prosthetic replacement therefore, to be replaced in the medical device without a surgical procedure.

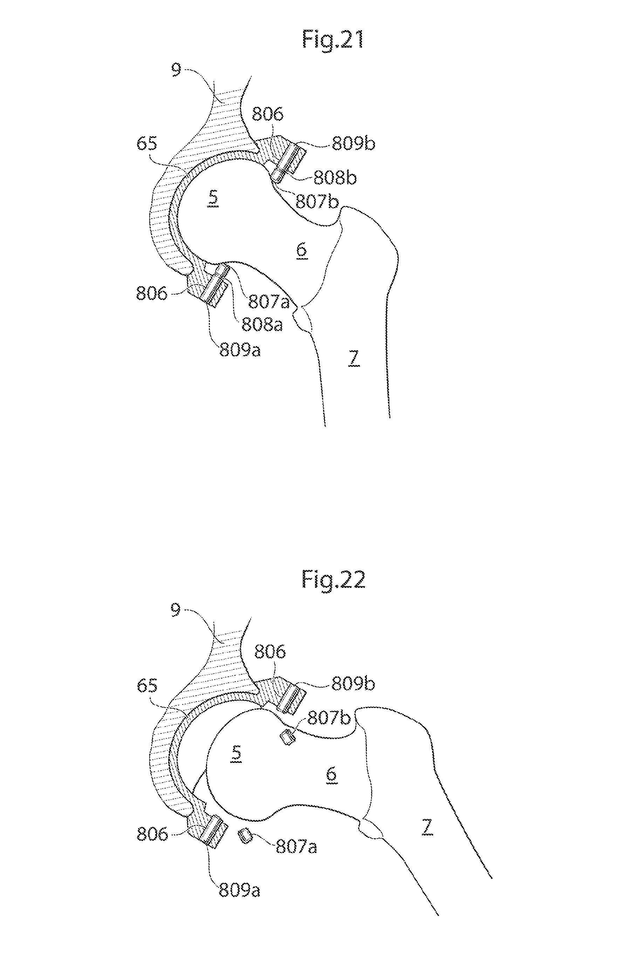

Fig 21 shows the medical device in an embodiment wherein the releasing members 801 comprises a rupture device 807, 808, 809 adapted to fail ata pre-determined strain. According to this embodiment the rupture device is a rupture pin 807, 808, 809 comprising a base part 809a,b fixated to the medical device and a rupture part 807a,b attached to the base part 809a,b through a weakened section 808a,b, in which section the rupture part 807a,b is detached from the base part 809a,b when a predetermined strain is placed on the rupture device in contact with the caput femur 5, or a prosthetic replacement therefore.

Fig. 22 shows the medical device according to the embodiment of fig. 21 when the rupture device has failed due to a pre-determined strain on the rupture device being exceeded. According to one embodiment, (not shown) the rupture parts 807a,b are secured to the base part through a security wire keeping rupture parts 807a,b in proximity to the base part 809a,b even after the failure of the rupture device.

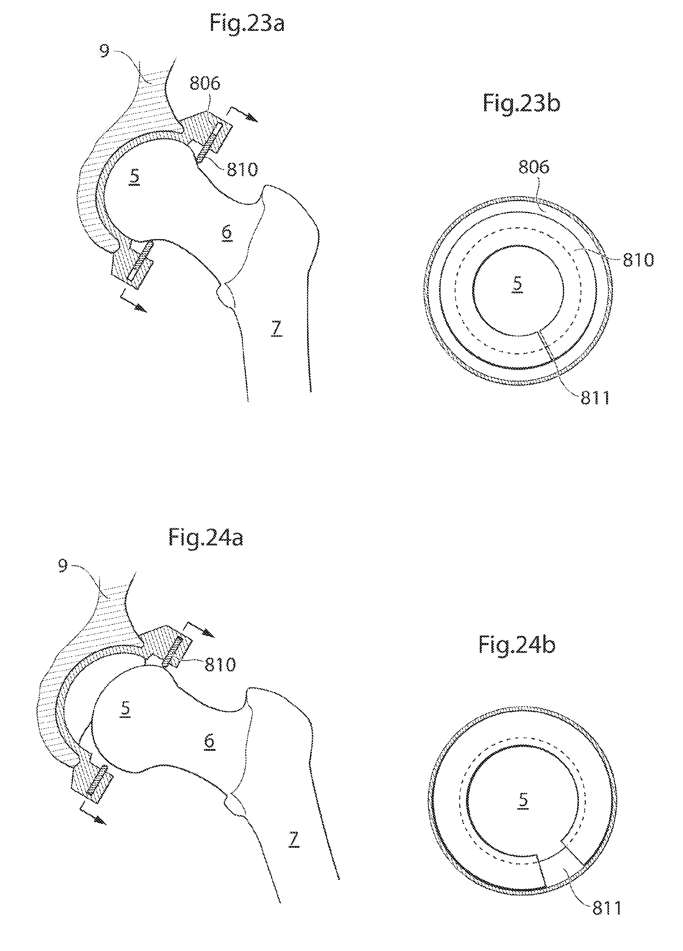

Fig 23a shows the medical device according to an embodiment where the medical device comprises a circular sleeve 806, in which an elastic or rupture band 810 is provided. The elastic or rupture band 810 is adapted to at least partly encircle the ball shaped caputfemur5, or prosthetic replacement therefore. When a pre-determined strain is placed on the elastic or rupture band 810 the circular opening encircling the caput femur 5, or a prosthetic replacement therefore, is expanded and the caput femur 5, or a prosthetic replacement therefore, is released from the medical device, to which it is held by means of the elastic band 610. In embodiments where the medical device comprises a rupture band 810 holding the caput femur 5, or a prosthetic replacement therefore, in the medical device, a weakened portion 811 of the band 810 fails and thus the circular opening encircling the caput femur 5, or a prosthetic replacement therefore, is expanded and the caput femur 5, or a prosthetic replacement therefore, is released from the medical device. In the embodiments where the band 810 is an elastic band 810 it is conceivable that the band 810 comprises an elastic part or section, or that the entire band 810 is made of an elastic material.

Fig. 23b shows the medical device in section when the elastic or rupturing band 810, holding the caput femur 5, or a prosthetic replacement therefore, is placed in a circular sleeve 806 in the medical device. An opening or weakened portion 811 is provided perpendicular to the circumference of the band 810.

Fig. 24a shows the medical device in a second state where the caput femur 5, or a prosthetic replacement therefore, is released from the connection with the medical device, after a pre-determined stain has been placed on the elastic or rupture band 810. As shown in fig. 24b, the gap or weakened part has been expanded, thereby allowing the caput femur, or a prosthetic replacement therefore, 5 to pass through the opening defined by the elastic or rupture band 810. The medical device could be adapted to non-invasively be able to change from the first state to the second state and from the second state to the first state, when a pre-determined strain is placed on the band 810, which enables the caput femur 5, or a prosthetic replacement therefore, to be replaced in the medical device without a surgical procedure.

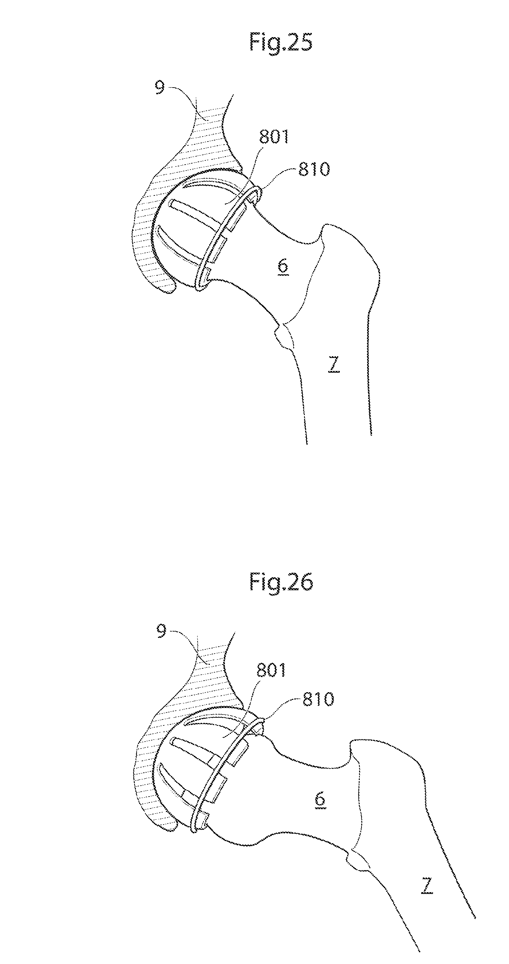

Fig. 25 shows the medical device according to an embodiment where the releasing member 801 comprises an elastic wing of the medical device, which is assisted by an elastic or rupture band 810 encircling the medical device by enclosing the caput femur 5, or a prosthetic replacement therefore, in the medical device passing beyond the point of the caputfemur5, or a prosthetic replacement therefore, having a largest cross-sectional distance. The elastic or rupture band 810 is held in place to the medical device by means of the band 810 being placed in a groove along the circumference of the medical device. However, said groove could be assisted or replaced by an adhesive or a mechanical fixation element

Fig. 26 shows the medical device when in its second state, in which the releasing member 801 releases the caput femur 5 or a prosthetic replacement therefore, from the medical device. In embodiments when the band 810 is an elastic band 810 it could be expanded, thereby enlarging the hole through which the caput femur 5, or a prosthetic replacement therefore, can pass. In embodiments where the band 810 is a rupture band, the band 810 fails and thereby the caput femur 5, or a prosthetic replacement therefore, is held in place solely by the releasing member 801 which is a part of the extending portion adapted to release the caput femur 5, or a prosthetic replacement therefore, at a pre-defined strain. The medical device could be adapted to non-invasively be able to change from the first state to the second state and from the second state to the first state, when a pre-determined strain is placed on the band 810 and/ or the releasing member 801, which enables the caput femur 5, ora prosthetic replacement therefore, to be replaced in the medical device without a surgical procedure.

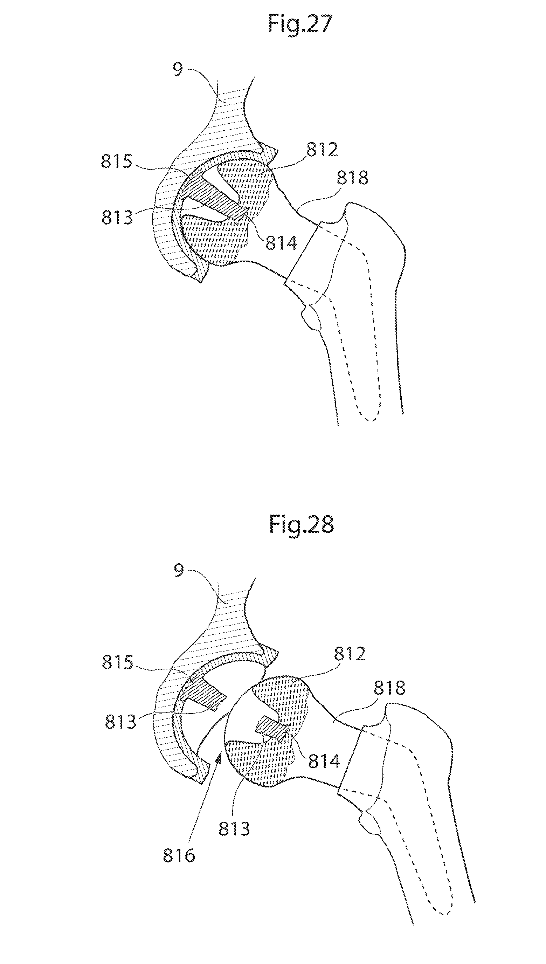

Fig. 27 shows the hip joint in section according to an embodiment where the caput femur 5, ora prosthetic replacement therefore, and collumfemur6 have been replaced with a prosthetic part 818 fixated to the femoral bone 7, either with bone cement, or without The prosthetic part 818 comprises a prosthetic caput femur 812 having a cavity 816 in which a rupture band 813 fixated to a fixation portion 814 of the prosthetic caput femur 812, and a fixating portion 815 of the medical device. The cavity 816 is adapted to enable the prosthetic caput femur 812 to perform normal functional hip movements inside the medical device. The rupture band 813 is adapted to hold the prosthetic caputfemur 812 to the medical device in a first state, and release the prosthetic caput femur 812 firom the medical device when a pre-determined strain is placed on the rupture band 813.

Fig. 28 shows the embodiment of the medical device according to fig. 27, in a second state in which the rupture band 813 has failed and thereby the prosthetic caput femur 812 is released from the medical device. The rupture band 813 could be fixated to a fixation portion 814 of the prosthetic caput femur 812, and/ or a fixating portion 815 of the medical device using: at least one screw, at least one pin, form fitting, welding, adhesive, pin, wire, a ball mounted into a bowl, a male portion of one part mounted into a female portion of the other part, a key introduced into a lock being portions of said parts, band, or other mechanical connecting members. The failing of the rupture band 813 is which could be caused by an abnormal movement of the hip joint, e.g. as the result of the patient falling.

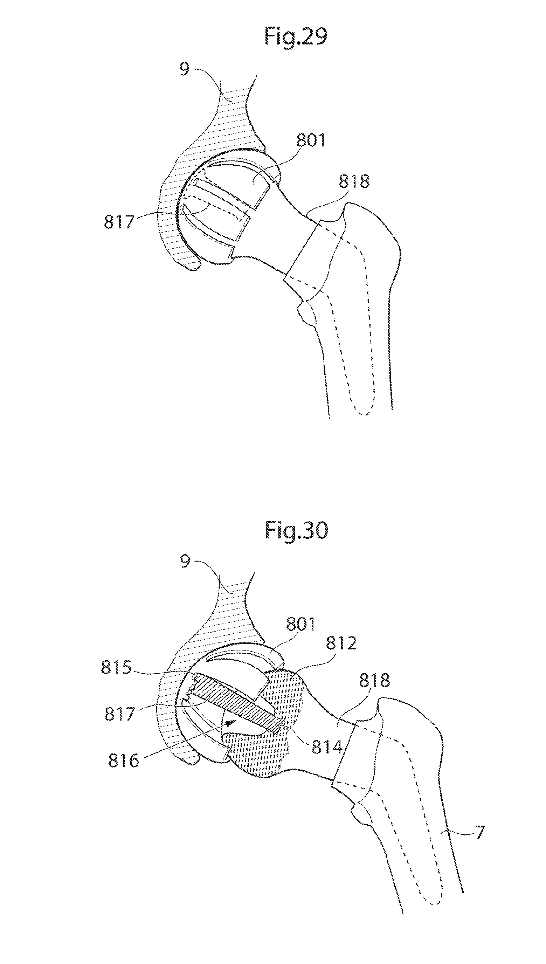

Fig. 29 shows a prosthetic part 818 according to an embodiment where the prosthetic part 818 is fixated to the femoral bone 7 and comprises a caput femur 812 comprising a cavity 816 adapted to enable the hip joint to perform functional hip joint movements while in a first state held to the medical device using an elastic bend 817 fixated to a fixation portion 814 of the prosthetic caput femur 812, and a fixating portion 815 of the medical device, and a releasing member 801 according to the embodiment shown in figs. 9 and 10. The combination of the releasing member 801 and the elastic band 817 is adapted to, in a first state hold the prosthetic part 818 to the medical device, and in a second state release the prosthetic part 818 from the medical device. According to another embodiment (not shown) the prosthetic partis held to the medical device solely using the elastic band 817, of course also supported by the remainder of the hip joint capsule and the affected muscles. Fig 29 shows a prosthetic caput femur 812 and a prosthetic acetabulum comprising the releasing member 801. A totally prosthetic embodiment is conceivable with regards to all of the embodiments of the releasing members disclosed herein. In any of these embodiments i.e. in embodiments where both the caput femur and the acetabulum are prosthetic parts, the prosthetic caput femur and prosthetic acetabulum could be pre-mounted when implanted in a patient such that one unit is implanted in the patient comprising the functioning hip joint

Fig. 30 shows the embodiment of the medical device according to fig. 29, in a second state in which the elastic band 817 is stretched such that the prosthetic part 818 is released from the medical device. The elastic band 817 could be fixated to a fixation portion 814 of the prosthetic caput femur 812, and/ or a fixating portion 815 of the medical device using: atleastone screw, at least one pin, form fitting, welding, adhesive, pin, wire, a ball mounted into a bowl, a male portion of one part mounted into a female portion of the other part, a key introduced into a lock being portions of said parts, band, or other mechanical connecting members. The failing of the rupture band 813 is preferably caused by an abnormal movement of the hip joint, e.g. as the result of the patient falling. The elastic band 817 could comprise an elastic part or section, which could be the entire elastic band 818, made from an elastic material, such as an elastic polymer material such as: a copolymer material such as polystyrene, poly(ethylene-butylene) orpolystyrene. It is also conceivable that the material is a polyurethane elastomeric material, polyamide elastomeric materials and polyester elastomeric materials elastic copolymer of ethylene and atleastone vinyl monomer such as, for example, vinyl acetates, unsaturated aliphatic monocarboxylic acids, and esters of such monocarboxylic acids. The elastic band 813 could comprise a barrier coating, which cannot be penetrated by body cells. Preferably, the barrier coating comprises a Parylene™ coating, or a biocompatible metal coating, such as gold, silver or titanium According to other embodiments the elastic band comprises a spring type member, a combination of metal and plastic materials, a combination of metal and carbon based material or a combination of carbon and plastic based material.

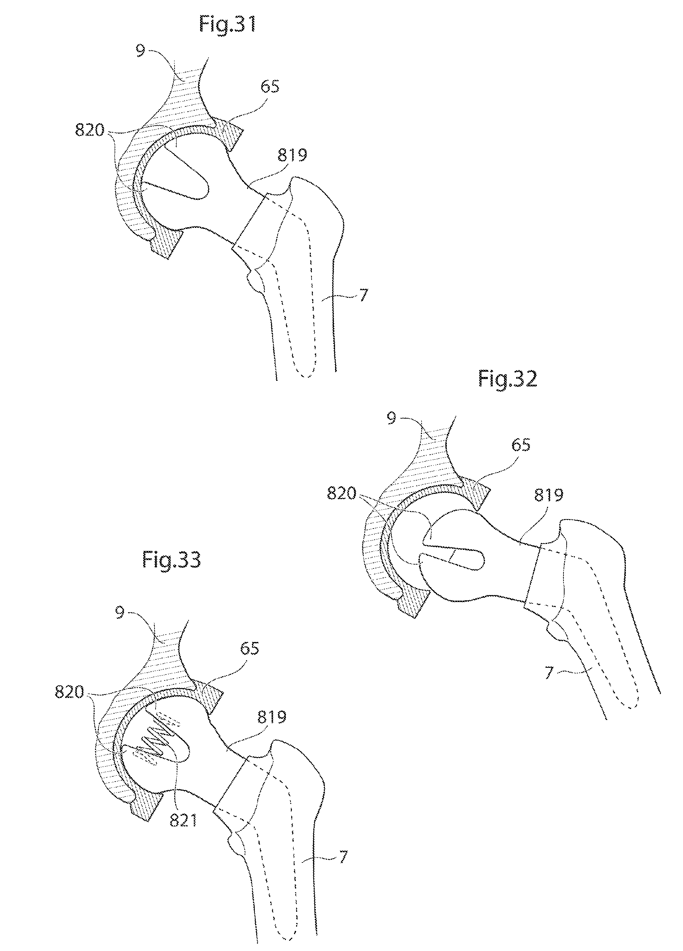

Fig. 31 shows the hip joint in section in an embodiment where the medical device comprises a prosthetic part 819 adapted to be fixated to the femoral bone 7. The prosthetic part comprises a prosthetic caput femur which is adapted to comprise elastic elements 820 which act as a releasing member holding the prosthetic caput femur inside of the medical device fixated to the pelvic bone. The elastic elements 820 of the prosthetic caput femur, is preferably made of an elastic material, which for example could be an elastomeric polymer material or an elastic metal material. It is conceivable that the elastic material comprises an outer layer in connection with the medical device which is adapted to resist the wear from the contact with the medical device. The elastic element is adapted to compress when a pre-determined strain is placed on the hip jointand thereby on the elastic elements 820. When the elastic elements 820 are compressed the prosthetic caput femur is released from the medical device.

Fig. 32 shows the medical device according to the embodiment shown in fig. 31, in a second state, in which the elastic element 820 has been compressed, following a predetermined strain being placed on the medical device. The medical device is thereby placed in a second state, in which the prosthetic caput femur is released from the medical device, wherein it has been held.

Fig. 33 shows an embodiment of the medical device in which the elastic elements 820 are further assisted by a spring 821 in connection with two elastic elements 820, the spring 821 is compressed alongside the elastic members 820, when a pre-determined strain is placed on the prosthetic part 819 comprising the prosthetic caput femur.

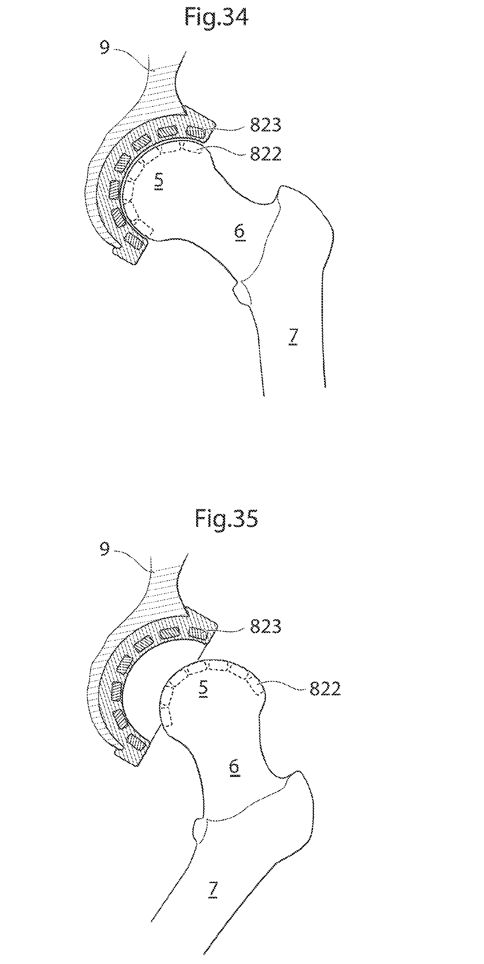

Fig. 34 shows the hip joint in section when a medical device for, in a first state, holding the caput femur 5, or a prosthetic replacement therefore, to the medical device, and in a second state releasing the caput femur 5, or a prosthetic replacement therefore from the medical device. The medical device is adapted to change from being in the first state to being in the second state at a pre-determined strain affecting the medical device by the connection with the pelvic bone 9 and the femoral bone 7, which reduced the risk of the patient fracturing the femoral bone 7 and/ or the pelvic bone 9. The medical device comprises magnets 823 or magnetic material 823 placed in the medical device, and magnets 822 or magnetic material 822 placed in the caput femur 5 or a prosthetic replacement therefore. According to one embodiment a magnet 823 is placed in the medical device its south pole directed towards the caput femur 5, or prosthetic replacement therefore, and a magnet822 placed in the caput femur 5, or prosthetic replacement therefore, having its north pole directed towards the medical device. However only one of the sides needs to be magnetic whereas the other side merely needs to comprise magnetic material. Any combination of north and south ends and magnets/ magnetic material is hence conceivable. The magnetic force described is adapted to hold the caput femur 5, or a prosthetic replacement therefore, in the medical device in normal use, enabling the hip joint to perform functional hip joint movements, and release the caput femur 5, or a prosthetic replacement therefore, from the medical device when a predetermined strain is exceeded.

Fig. 35 shows the medical device according to the embodiment of fig. 30 in the second state, in which the caput femur 5, or a prosthetic replacement therefore, is released from the medical device as a result of a pre-determined level of strain being exceeded.

The medical device according to any of the embodiments could comprise at least one material selected firom a group consisting of: polytetrafluoroethylene (PIFE), perfluoroalkoxy (PFA) and fluorinated ethylene propylene (FEP). It is furthermore conceivable that the material comprises a metal alloy, such as cobalt-chromium-molybdenum or titanium or stainless steel, or polyethylene, such as cross-linked polyethylene or gas sterilized polyethylene. The use of ceramic material is also conceivable, in the contacting surfaces or the entire medical device such as zirconium or zirconium dioxide ceramics or alumina ceramics. The part of the medical device in contact with human bone for fixation of the medical device to human bone could comprise a poorhouse structure which could be a porous micro or nano-structure adapted to promote the growth-in of human bone in the medical device for fixating the medical device. The porous structure could be achieved by applying a hydroxy-apatite (HA) coating, or a rough open-pored titanium coating, which could be produced by air plasma spraying, a combination comprising a rough open-pored titanium coating and a HA top layer is also conceivable. The contacting parts could be made of a self lubricated material such as a waxy polymer, such as PTFE, PFA, FEP, PE and UHMWPE, or a powder metallurgy material which could be infused with a lubricant, which preferably is a biocompatible lubricant such as a Hyaluronic acid derivate. It is also conceivable that the material of contacting parts or surfaces of the medical device herein is adapted to be constantly or intermittently lubricated. According to some embodiments the parts or portions of the medical device could comprise a combination of metal materials and/ or carbon fibers and/ or boron, a combination of metal and plastic materials, a combination of metal and carbon based material, a combination of carbon and plastic based material, a combination of flexible and stiff materials, a combination of elastic and less elastic materials, Corian or acxylic polymers. Please note that any embodiment or part of embodiment could be combined in any way. All examples herein should be seen as part of the general description and therefore possible to combine in any way in general terms.

## Claims

1. A medical device for implantation in a hip joint of a patient, wherein said medical device is adapted to be fixated to the pelvic bone (9) of the patient, and wherein said medical device comprises an inner (1827) and an outer (1828) surface, the inner surface comprises an equator line (1821), being the largest circular circumference of said inner surface, wherein a contacting portion of said inner surface is spherical and adapted to face the centre of the hip joint when said medical device is implanted, and wherein said medical device is adapted to receive a caput femur or a prosthetic caput femur having a spherical portion, wherein
a) said medical device comprises at least one extending portion (1823) extending beyond said circular equator line and being adapted to clasp the caput femur (5), or prosthetic caput femur, for restraining said caput femur, or prosthetic caput femur in said medical device,
b) said medical device is adapted to release the caput femur or prosthetic caput femur from said medical device when a predetermined strain is placed on said medical device,
c) **characterized in that** the extending portion is placed dorsal to the proximal-distal axis Y, when implanted, and wherein the medical device comprises a second portion not extending beyond said circular equator and extending along at least 1/2 of said circular equator line, and extending frontal to the proximal-distal axis Y, such that the motion range of the hip joint is limited in a non-restrictive way.

2. The medical device according to claim 1, wherein said at least one extending portion adapted to clasp the caput femur, comprises an elastic portion.

3. The medical device according to claim 2, wherein said elastic portion comprises an elastic material.

4. The medical device according to any one of the preceding claims, wherein said at least one extending portion comprises a movable portion adapted to clasp the caput femur, and further adapted to move such that said movable portion releases the caput femur or prosthetic caput femur from said medical device, when a predetermined strain is placed on said medical device.

5. The medical device according to any one of the preceding claims, wherein said at least one extending portion comprises a magnet (822) adapted to hold the caput femur in the medical device.

6. The medical device according to any one of the preceding claims, wherein said at least one extending portion comprises a rupture device (807; 808; 809; 813) adapted to fail at a pre-determined strain.

7. The medical device according to any one of the preceding claims, wherein said at least one extending portion (802; 805) is adapted to slide against the caput femur.

8. The medical device according to any one of the preceding claims, wherein said at least one extending portion (802; 805) is adapted to roll against the caput femur.

9. The medical device according to any one of the preceding claims, wherein said at least one extending portion extends longitudinally along said equator line, at least one of:
a) dorsal to a lateral-medial axis (X) of pelvis,
b) dorsal to the lateral-medial axis and proximal to the lateral-medial axis of pelvis,
c) dorsal to the lateral-medial axis and distal to the lateral-medial axis of pelvis, and
d) dorsal to the lateral-medial axis and distal to the lateral-medial axis of the pelvis, and one extending portion extends dorsal to the lateral-medial axis and proximal to the lateral-medial axis of the pelvis.

10. The medical device according to any one of the preceding claims, wherein said at least one extending portion extends longitudinally along said equator line, at least one of:
a) in the proximal quadrant (1811) thereof,
b) in the distal (1813) quadrant thereof,
c) in the distal and proximal quadrant thereof,
d) in the proximal and dorsal quadrant (814) thereof,
e) in the distal and dorsal quadrant thereof, and
f) in the distal, dorsal and proximal quadrant thereof.

11. The medical device according to any one of the preceding claims, wherein said extending portion longitudinally extends at least one of:
a) along at least 1/2 of said circular equator line,
b) along at least 1/3 of said circular equator line,
c) along at least 1/4 of said circular equator line.
d) along at least zu of said circular equator line, and
e) along at least 1/10 of said circular equator line, and said second portion longitudinally extends along at least % of said circular equator line.

12. The medical device according to any one of the preceding claims, wherein at least two first portions of said medical device are extending portions, extending beyond said circular equator line, and wherein one of said extending portions extends further than the other extending portion.

13. The medical device according to any one of the preceding claims, wherein said medical device further comprises at least one hole, and wherein said at least one hole is adapted to receive a fixating member (1830), for fixating said medical device to the pelvic bone.

14. The medical device according to any one of the preceding claims, wherein said medical device comprise a prosthetic caput femur, and a prosthetic acetabulum pre-mounted to a functional hip joint for implantation in the hip joint of a patient.

15. The medical device according to any one of the preceding claims, wherein said at least one of extending portion, when implanted, is adapted to be placed such as to restrict the motion range of the hip joint, and wherein said at least one extending portion Is adapted to be placed or shaped such that at least one of adduction, abduction, flexion, extension, a combination of flexion and adduction or abduction, a combination of extension and adduction or abduction, rotation in, rotation out, and any combination of rotation in or out and the other described movements, is restricted more degrees from maximal movement than any of the other.

## Patentansprüche

1. Medizinische Vorrichtung für die Implantation in ein Hüftgelenk eines Patienten, wobei die medizinische Vorrichtung für die Fixierung an den Beckenknochen (9) des Patienten ausgelegt ist und wobei die medizinische Vorrichtung eine innere (1827) und eine äußere (1828) Oberfläche aufweist, die innere Oberfläche eine Äquatorlinie (1821) umfasst, die der größte Kreisumfang der inneren Oberfläche ist, wobei ein kontaktierender Teil der inneren Oberfläche sphärisch ist und dafür ausgelegt ist, zu der Mitte des Hüftgelenks zu weisen, wenn die medizinische Vorrichtung implantiert ist, und wobei die medizinische Vorrichtung dafür ausgelegt ist, einen Oberschenkelkopf oder eine Oberschenkelkopfprothese mit einem sphärischen Teil aufzunehmen, wobei
a) die medizinische Vorrichtung wenigstens einen verlängerten Teil (1823) umfasst, der über die kreisförmige Äquatorlinie verlängert ist und dafür ausgelegt ist, den Oberschenkelkopf (5) oder die Oberschenkelkopfprothese zu umklammern, um den Oberschenkelkopf oder die Oberschenkelkopfprothese in der medizinischen Vorrichtung festzuhalten,
b) die medizinische Vorrichtung dafür ausgelegt ist, den Oberschenkelkopf oder die Oberschenkelkopfprothese aus der medizinischen Vorrichtung freizugeben, wenn eine vorbestimmte Belastung an die medizinische Vorrichtung angelegt wird,
c) **dadurch gekennzeichnet, dass** der verlängerte Teil, wenn implantiert, dorsal bezogen auf die proximal-distale Achse Y angeordnet ist und wobei die medizinische Vorrichtung einen zweiten Teil umfasst, der nicht über den kreisförmigen Äquator verlängert ist und sich über wenigstens 1/2 der kreisförmigen Äquatorlinie erstreckt und sich frontal bezogen auf die proximal-distale Achse Y erstreckt, so dass der Bewegungsbereich des Hüftgelenks auf nicht-einschränkende Weise beschränkt ist.

2. Medizinische Vorrichtung gemäß Anspruch 1, wobei der wenigstens eine verlängerte Teil, der zum Umklammern des Oberschenkelkopfs ausgelegt ist, einen elastischen Teil umfasst.

3. Medizinische Vorrichtung gemäß Anspruch 2, wobei der elastische Teil ein elastisches Materiat umfasst.

4. Medizinische Vorrichtung gemäß einem der vorstehenden Ansprüche, wobei der wenigstens eine verlängerte Teil einen beweglichen Teil umfasst, der dafür ausgelegt ist, den Oberschenkelkopf zu umklammern, und ferner dafür ausgelegt ist, sich so zu bewegen, dass der bewegliche Teil den Oberschenkelkopf oder die Oberschenkelkopfprothese aus der aus der medizinischen Vorrichtung freigibt, wenn eine vorbestimmte Belastung an die medizinische Vorrichtung angelegt wird.

5. Medizinische Vorrichtung gemäß einem der vorstehenden Ansprüche, wobei der wenigstens eine verlängerte Teil einen Magneten (822) umfasst, der dafür ausgelegt ist, den Oberschenkelkopf in der medizinischen Vorrichtung zu halten.

6. Medizinische Vorrichtung gemäß einem der vorstehenden Ansprüche, wobei der wenigstens eine verlängerte Teil eine Bruchvorrichtung (807; 808; 809; 813) umfasst, die dafür ausgelegt ist, bei einer vorbestimmten Belastung zu brechen.

7. Medizinische Vorrichtung gemäß einem der vorstehenden Ansprüche, wobei der wenigstens eine verlängerte Teil (802; 805) dafür ausgelegt ist, gegen den Oberschenkelkopf zu gleiten.

8. Medizinische Vorrichtung gemäß einem der vorstehenden Ansprüche wobei der wenigstens eine verlängerte Teil (802; 805) dafür ausgelegt ist, gegen den Oberschenkelkopf zu rollen.

9. Medizinische Vorrichtung gemäß einem der vorstehenden Ansprüche, wobei sich der wenigstens eine verlängerte Teil (802; 805) gemäß wenigstens einem von folgendem longitudinal entlang der Äquatorlinie erstreckt:
a) dorsal bezogen auf eine lateral-mediale Achse (X) des Beckens,
b) dorsal bezogen auf die lateral-mediale Achse und proximal bezogen auf die lateral-mediale Achse des Beckens,
c) dorsal bezogen auf eine lateral-mediale Achse und distal bezogen auf die lateral-mediale Achse des Beckens und
d) dorsal bezogen auf die lateral-mediale Achse und distal bezogen auf die lateral-mediale Achse des Beckens und sich ein verlängerter Teil dorsal bezogen auf die lateral-mediale Achse und proximal bezogen auf die lateral-mediale Achse des Beckens erstreckt.

10. Medizinische Vorrichtung gemäß einem der vorstehenden Ansprüche, wobei sich der wenigstens eine verlängerte Teil gemäß wenigstens einem von folgendem longitudinal entlang der Äquatorlinie erstreckt:
a) in dem proximalen Quadranten (1811) davon,
b) in dem distalen Quadranten (1813) davon,
c) in dem distalen und proximalen Quadranten davon,
d) in dem proximalen und dorsalen Quadranten (814) davon,
e) in dem distalen und dorsalen Quadranten davon und
f) in dem distalen, dorsalen und proximalen Quadranten davon.

11. Medizinische Vorrichtung gemäß einem der vorstehenden Ansprüche, wobei sich der verlängerte Teil entlang wenigstens einem von folgendem longitudinal erstreckt:
a) entlang von wenigstens 1/2 der kreisförmigen Äquatorlinie,
b) entlang von wenigstens 1/3 der kreisförmigen Äquatorlinie,
c) entlang von wenigstens 1/4 der kreisförmigen Äquatorlinie,
d) entlang von wenigstens 1/10 der kreisförmigen Aquatorlinie und
e) entlang von wenigstens 1/10 der kreisförmigen Äquatorlinie und sich der zweite Teil entlang von wenigstens 1/4 der kreisförmigen Äquatorlinie longitudinal erstreckt.

12. Medizinische Vorrichtung gemäß einem der vorstehenden Ansprüche, wobei wenigstens zwei Teile der medizinischen Vorrichtung verlängerte Teile sind, die über die kreisförmige Äquatorlinie verlängert sind, und wobei sich einer der verlängerten Teile weiter als der andere verlängerte Teil erstreckt.

13. Medizinische Vorrichtung gemäß einem der vorstehenden Ansprüche, wobei die medizinische Vorrichtung ferner wenigstens ein Loch umfasst und wobei das wenigstens eine Loch dafür ausgelegt ist, ein Fixierelement (1830) zum Fixieren der medizinischen Vorrichtung an den Beckenknochen aufzunehmen.

14. Medizinische Vorrichtung gemäß einem der vorstehenden Ansprüche, wobei die medizinische Vorrichtung eine Oberschenkelkopfprothese und eine Hüftgelenkpfannenprothese umfasst, die zu einem funktionellen Hüftgelenk für die Implantation in das Hüftgelenk eines Patienten vormontiert sind.

15. Medizinische Vorrichtung gemäß einem der vorstehenden Ansprüche, wobei der wenigstens eine verlängerte Teil, wenn implantiert, dafür ausgelegt ist, platziert zu werden, um den Bewegungsbereich des Hüftgelenks zu beschränken, und wobei der wenigstens eine verlängerte Teil dafür ausgelegt ist, so platziert zu werden oder geformt zu sein, dass wenigstens eines von Adduktion, Abduktion, Flexion, Extension, eine Kombination von Flexion und Adduktion oder Abduktion, eine Kombination von Extension und Adduktion oder Abduktion, Rotation einwärts, Rotation auswärts und jede Kombination von Rotation einwärts oder auswärts und den anderen beschriebenen Bewegungen um mehr Grad von der maximalen Bewegung beschränkt ist als jedes der anderen.

## Revendications

1. Dispositif médical à implanter dans l'articulation de la hanche d'un patient, dans lequel ledit dispositif médical est apte à être fixé à l'os pelvien (9) du patient, et dans lequel ledit dispositif médical présente une surface intérieure (1827) et extérieure (1828), la surface intérieure comprenant une ligne équinoxiale (1821) qui correspond à la plus grande circonférence circulaire de ladite surface intérieure, dans lequel une partie de contact de ladite surface intérieure est sphérique et apte à se trouver en face du centre de l'articulation de la hanche lorsque ledit dispositif médical est implanté, et dans lequel ledit dispositif médical est apte à recevoir une tête de fémur ou une tête de fémur prothétique présentant une partie sphérique, dans lequel:
a) ledit dispositif médical comprend au moins une partie d'extension (1823) qui s'étend au-delà de ladite ligne équinoxiale circulaire et qui est apte à accrocher la tête de fémur (5), ou une tête de fémur prothétique, afin de caler ladite tête de fémur ou tête de fémur prothétique dans ledit dispositif médical,
b) ledit dispositif médical est apte à libérer la tête de fémur ou la tête de fémur prothétique dudit dispositif médical lorsqu'une contrainte prédéterminée est exercée sur ledit dispositif médical,
c) **caractérisé en ce que** la partie d'extension est placée de façon dorsale à l'axe proximal-distal Y, lorsqu'il est implanté, et dans lequel le dispositif médical comprend une deuxième partie qui ne s'étend pas au-delà dudit équateur circulaire et qui s'étend le long d'au moins 1/2 de ladite ligne équinoxiale circulaire, et qui s'étend de façon frontale à l'axe proximal-distal Y, de telle sorte que l'amplitude de mouvement de l'articulation de la hanche soit limitée d'une manière non restrictive.

2. Dispositif médical selon la revendication 1, dans lequel ladite au moins une partie d'extension apte à accrocher la tête de fémur comprend une partie élastique.

3. Dispositif médical selon la revendication 2, dans lequel ladite partie élastique comprend une matière élastique.

4. Dispositif médical selon l'une quelconque des revendications précédentes, dans lequel ladite au moins une partie d'extension comprend une partie mobile apte à accrocher la tête de fémur, et apte également à se déplacer de telle sorte que ladite partie mobile libère la tête de fémur ou la tête de fémur prothétique dudit dispositif médical lorsqu'une contrainte prédéterminée est exercée sur ledit dispositif médical.

5. Dispositif médical selon l'une quelconque des revendications précédentes, dans lequel ladite au moins une partie d'extension comprend un aimant (822) apte à maintenir la tête de fémur dans le dispositif médical.

6. Dispositif médical selon l'une quelconque des revendications précédentes, dans lequel ladite au moins une partie d'extension comprend un dispositif de rupture (807; 808; 809; 813) apte à se rompre sous une contrainte prédéterminée.

7. Dispositif médical selon l'une quelconque des revendications précédentes, dans lequel ladite au moins une partie d'extension (802; 805) est apte à glisser contre la tête de fémur.

8. Dispositif médical selon l'une quelconque des revendications précédentes, dans lequel ladite au moins une partie d'extension (802; 805) est apte à rouler contre la tête de fémur.

9. Dispositif médical selon l'une quelconque des revendications précédentes, dans lequel ladite au moins une partie d'extension s'étend de façon longitudinale le long de ladite ligne équinoxiale, d'au moins une façon parmi:
a) dorsale à un axe latéral-médial (X) du pelvis,
b) dorsale à l'axe latéral-médial et proximale à l'axe latéral-médial du pelvis,
c) dorsale à l'axe latéral-médial et distale à l'axe latéral-médial du pelvis,
d) dorsale à l'axe latéral-médial et distale à l'axe latéral-médial du pelvis, et une partie d'extension s'étend de façon dorsale à l'axe latéral-médial et proximale à l'axe latéral-médial du pelvis.

10. Dispositif médical selon l'une quelconque des revendications précédentes, dans lequel ladite au moins une partie d'extension s'étend de façon longitudinale le long de ladite ligne équinoxiale, d'au moins une façon parmi:
a) dans le quadrant proximal (1811) de celle-ci,
b) dans le quadrant distal (1813) de celle-ci,
c) dans le quadrant distal et proximal de celle-ci,
d) dans le quadrant proximal et distal (814) de celle-ci,
e) dans le quadrant distal et dorsal de celle-ci, et
f) dans le quadrant distal, dorsal et proximal de celle-ci.

11. Dispositif médical selon l'une quelconque des revendications précédentes, dans lequel ladite au moins une partie d'extension s'étend de façon longitudinale d'au moins une façon parmi:
a) le long d'au moins 1/2 de ladite ligne équinoxiale circulaire,
b) le long d'au moins 1/3 de ladite ligne équinoxiale circulaire,
c) le long d'au moins 1/4 de ladite ligne équinoxiale circulaire,
d) le long d'au moins 1/10 de ladite ligne équinoxiale circulaire, et
e) le long d'au moins 1/10 de ladite ligne équinoxiale circulaire, et ladite deuxième partie s'étend de façon longitudinale le long d'au moins 1/4 de ladite ligne équinoxiale circulaire.

12. Dispositif médical selon l'une quelconque des revendications précédentes, dans lequel au moins deux premières parties dudit dispositif médical sont des parties d'extension, qui s'étendent au-delà de ladite ligne équinoxiale circulaire, et dans lequel une desdites parties d'extension s'étend plus loin que l'autre partie d'extension.

13. Dispositif médical selon l'une quelconque des revendications précédentes, dans lequel ledit dispositif médical comporte en outre au moins un trou, et dans lequel ledit au moins un trou est apte à recevoir un élément de fixation (1830) pour fixer ledit dispositif médical à l'os pelvien.

14. Dispositif médical selon l'une quelconque des revendications précédentes, dans lequel ledit dispositif médical comprend une tête de fémur prothétique, et un acétabulum prothétique qui est pré-monté sur une articulation de hanche fonctionnelle à implanter dans la hanche d'un patient.

15. Dispositif médical selon l'une quelconque des revendications précédentes, dans lequel ladite au moins une partie d'extension, lorsqu'elle est implantée, est apte à être placée de manière à limiter l'amplitude du mouvement de l'articulation de la hanche, et dans lequel ladite au moins une partie d'extension est apte à être placée ou configurée de telle sorte qu'au moins une adduction, une abduction, une flexion, une extension, une combinaison de flexion et d'adduction ou d'abduction, une combinaison d'extension et d'adduction ou d'abduction, une rotation intérieure, une rotation extérieure, ainsi que toute combinaison de rotation intérieure ou extérieure et des autres mouvements décrits, soit limitée par rapport à son mouvement maximal dans une plus grande mesure que l'un quelconque des autres mouvements.
